# EUROPÄISCHE PATENTSCHRIFT

(11) **EP 3 263 077 B1**
(45) Veröffentlichungstag und Bekanntmachung des Hinweises auf die Patenterteilung: **24.03.2021**
(21) Anmeldenummer: 17172442.0
(22) Anmeldetag: 22.08.2003
(51) Int. Cl.: A61F 9/008

(54) **VORRICHTUNG ZUR BEHANDLUNG EINES GEWEBES**
DEVICE FOR TREATING A TISSUE
DISPOSITIF DE TRAITEMENT D'UN TISSU

(30) Priorität: 23.08.2002 DE 10239213; 23.05.2003 DE 10323422
(43) Veröffentlichungstag der Anmeldung: 03.01.2018
(62) Teilanmeldung aus: 03750427.1
(73) Patentinhaber: Carl Zeiss Meditec AG, 07745 Jena (DE)
(72) Erfinder: KEMPE, Michael, 07751 Jena (DE); STREHLE, Markus, 07751 Jena (DE); MÜHLHOFF, Dirk, 07751 Jena (DE); GERLACH, Mario, 16548 Glienicke-Nordbahn (DE); STICKER, Markus, 07749 Jena (DE); BISCHOFF, Mark, 07749 Jena (DE); DICK, Manfred, 07926 Gefell (DE); BERGT, Michael, 99425 Weimar (DE)
(74) Vertreter: Patentanwälte Geyer, Fehners & Partner mbB

(56) Entgegenhaltungen:
- EP-A1- 1 232 734
- WO-A1-01/91661
- DE-C1- 19 635 998
- US-B1- 6 454 761

## Beschreibung

Die Erfindung bezieht sich auf eine Vorrichtung zur Behandlung von transparentem Gewebe eines Auges.

Behandlungs-Laserstrahlung wird in laserchirurgischen Verfahren insbesondere für augenchirurgische Eingriffe angewendet. Ein Anwendungsgebiet ist die Koagulation an der Netzhaut, wie sie z. B. Gegenstand der DE 19635998 C1 ist. Ein anderes Gebiet ist die Behandlung von transparentem Gewebe. Dabei wird die Behandlungs-Laserstrahlung innerhalb des Gewebes, d.h. unterhalb der Gewebeoberfläche, derart fokussiert, dass ein optischer Durchbruch im Gewebe ausgelöst wird. Die Behandlungs-Laserstrahlung wirkt durch Photodisruption oder -ablation.

Im Gewebe laufen zeitlich hintereinander mehrere Prozesse ab, die durch die Behandlungs-Laserstrahlung initiiert werden. Überschreitet die Leitungsdichte der Strahlung einen Schwellwert, kommt es zu einem optischen Durchbruch, der im Gewebe eine Plasmablase erzeugt. Diese Plasmablase wächst nach Entstehen des optischen Durchbruches durch sich ausdehnende Gase. Wird der optische Durchbruch nicht aufrechterhalten, so wird das in der Plasmablase erzeugte Gas vom umliegenden Gewebe aufgenommen, und die Blase verschwindet wieder. Dieser Vorgang dauert allerdings sehr viel länger, als die Entstehung der Blase selbst. Wird ein Plasma an einer Gewebegrenzfläche erzeugt, die durchaus auch innerhalb einer Gewebestruktur liegen kann, so erfolgt ein Gewebeabtrag von der Grenzfläche. Man spricht deshalb dann von Photoablation. Bei einer Plasmablase, die vorher verbundene Gewebeschichten trennt, ist üblicherweise von Photodisruption die Rede. Der Einfachheit halber werden all solche Prozesse hier unter dem Begriff optischer Durchbruch zusammengefasst, d.h. dieser Begriff schließt nicht nur den eigentlichen optischen Durchbruch sondern auch die daraus resultierenden Wirkungen im Gewebe ein.

Für eine hohe Genauigkeit eines laserchirurgischen Verfahrens ist es unumgänglich, eine hohe Lokalisierung der Wirkung der Behandlungs-Laserstrahlen zu gewährleisten und Kollateralschäden in benachbartem Gewebe möglichst zu vermeiden. Es ist deshalb im Stand der Technik üblich, die Behandlungs-Laserstrahlung gepulst anzuwenden, so dass der zur Auslösung eines optischen Durchbruchs nötige Schwellwert für die Leistungsdichte der Behandlungs-Laserstrahlung nur während den einzelnen Pulsen überschritten wird. Die US 5.984.916 zeigt diesbezüglich deutlich, dass der räumliche Bereich des optischen Durchbruchs (in diesem Fall der erzeugten Wechselwirkung) stark von der Pulsdauer abhängt. Eine hohe Fokussierung des Laserstrahls in Kombination mit sehr kurzen Pulsen erlaubt es damit, den optischen Durchbruch sehr punktgenau in einem Gewebe einzusetzen.

Der Einsatz von gepulster Behandlungs-Laserstrahlung hat sich in der letzten Zeit besonders zur laserchirurgischen Fehlsichtigkeitskorrektur in der Ophthalmologie durchgesetzt. Fehlsichtigkeiten des Auges rühren oftmals daher, dass die Brechungseigenschaften von Hornhaut und Linse keine ordnungsgemäße Fokussierung auf der Netzhaut bewirken. Bei Kurzsichtigkeit (auch als Myopie bezeichnet) liegt bei entspanntem Auge der Fokus vor der Netzhaut, bei Fernsichtigkeit (auch als Hyperopie bezeichnet) ist der Fokus dagegen hinter der Netzhaut.

Die erwähnte US 5.984.916 sowie die US 6.110.166 beschreiben Verfahren zur Fehlsichtigkeitskorrektur mittels geeigneter Erzeugung optischer Durchbrüche, so dass im Endeffekt die Brechungseigenschaften der Hornhaut gezielt beeinflusst werden. Eine Vielzahl von optischen Durchbrüchen wird so aneinander gesetzt, dass innerhalb der Hornhaut des Auges ein linsenförmiges Teilvolumen isoliert wird. Das vom übrigen Hornhautgewebe getrennte linsenförmige Teilvolumen wird dann mittels eines seitlich öffnenden Schnittes aus der Hornhaut herausgenommen. Die Gestalt des Teilvolumens ist so gewählt, dass nach Entnahme die Brechungseigenschaften der Hornhaut so geändert sind, dass die erwünschte Fehlsichtigkeitskorrektur bewirkt ist.

Zur Isolierung des Teilvolumens ist es natürlich unumgänglich, die optischen Durchbrüche an vorbestimmten Stellen zu erzeugen. Die US 5.984.916 beschreibt entsprechende Sensoren, die Querschnitt sowie Lage und Intensität des Behandlungs-Laserstrahls erfassen und eine entsprechende Steuereinrichtung speisen, die den Laserbehandlungsstrahl so beeinflusst, dass am gewünschten Zielpunkt ein gewünschter optischer Durchbruch erreicht wird.

Die EP 1 232 734 A1 schlägt dagegen vor, einen Wellenfrontsensor zu verwenden, um die Lage eines optischen Durchbruches, der in einem Auge erzeugt wurde, zu bestimmten. In der Veröffentlichung ist diesbezüglich davon die Rede, dass die Blasengröße mit dem Wellenfrontsensor unter Verwendung "relativ gut bekannter Wellenfronttechniken" gemessen werden kann. Über die Art und Weise wie die Messung erfolgen könnte, schweigt sich die Schrift leider aus. Sie vermittelt keine technische Lehre, sondern bleibt aufgabenhaft. Da ein Wellenfrontsensor aber bekanntermaßen in der Lage ist, eine Verzerrung einer Wellenfront festzustellen, wäre es denkbar, die in EP 1 232 734 A1 aufgabenhaft genannte Zielsetzung dadurch zu erreichen, dass mit einem Wellenfrontsensor eine Verformung der Hornhautoberfläche detektiert wird, die von einer im Inneren der Hornhaut erzeugten Blase hervorgerufen wurde. Bei einem solchen denkbaren Ansatz wäre es jedoch zu erwarten, dass die Messgenauigkeit mit abnehmender Blasengröße stark sinkt, da eine kleinere Blase natürlich auch zu einer stark verminderten Verformung der Hornhautvorderfläche führen wird. Darüber hinaus wäre zu erwarten, dass bei einer tiefer gelegenen Blase der durch diese Art der Messung angezeigte Blasendurchmesser geringer ausfällt, als bei einer höher gelegenen Blase gleicher Größe. Mit einem solchen Ansatz müsste also mit einem von der Tiefenlage der Blase abhängigen Messfehler gerechnet werden.

Die Genauigkeit, mit der Behandlungs-Laserstrahlung z.B. zur Fehlsichtigkeitskorrektur am Auge eines Patienten appliziert werden kann, hat natürlich direkte Auswirkung auf die Qualität des Ergebnisses, im genannten Beispiel auf die Qualität einer optischen Korrektur. Es ist deshalb Aufgabe der Erfindung, eine Vorrichtung zur Messung eines optischen Durchbruches zu schaffen, mit der eine gesteigerte Zielgenauigkeit der Wirkung einer Behandlungslaserstrahlung erreicht werden kann.

Die Erfindung ist in Anspruch 1 definiert.

Mit der Erfindung kann ein Gewebe sowohl diagnostiziert als auch dreidimensional vermessen und bei einem zur Behandlung vorgesehenen Gewebe auch behandelt werden. Dazu ist vorgesehen, ein Verfahren zur Vermessung eines transparenten oder halbtransparenten Gewebes, wobei eine Beleuchtungs-Laserstrahlung in einem Fokuspunkt im Gewebe fokussiert und die Lage des Fokuspunktes im Gewebe verändert wird, wozu eine veränderliche Ablenkung der Beleuchtungs-Laserstrahlung vorgenommen wird, und wobei durch die Fokussierung induzierte, gewebespezifische Signale detektiert und Messpunkten zugeordnet werden, deren Ort im Gewebe jeweils durch die bestimmte Lage des Fokuspunktes definiert ist, und wobei Messpunkte herausgefiltert und dadurch Lagen von Grenzschichten und/oder Einschlüssen im Gewebe bestimmt werden.

Dieses Verfahren kann besonders vorteilhaft realisiert werden mit einer Vorrichtung mit einer Laserstrahlungsquelle, einer Ablenkeinrichtung, einer Fokussiereinrichtung und einer Detektoreinrichtung sowie einer Steuereinrichtung, welche die Laserstrahlungsquelle, die Ablenkeinrichtung und die Fokussiereinrichtung so ansteuert, dass von der Laserstrahlungsquelle abgegebene Beleuchtungs-Laserstrahlung von der Ablenkeinrichtung und der Fokussiereinrichtung nacheinander in mehrere Fokuspunkte innerhalb des Gewebes fokussiert wird, wobei die Detektoreinrichtung durch die Fokussierung induzierte, gewebespezifische Signale an die Steuereinrichtung abgibt und die Steuereinrichtung die Signale Messpunkten zuordnet, deren Ort im Gewebe jeweils durch die Lage des Fokuspunktes definiert ist, und Messpunkte herausfiltert und dadurch Lagen von Grenzschichten und/oder Einschlüssen im Gewebe bestimmt.

Die herausgefilterten Messpunkte können besonders vorteilhaft verwendet werden, um Zielpunkte für eine nachfolgende Behandlung des Gewebes mittels im Gewebe fokussierter Behandlungslaserstrahlung zu erzeugen. Es ist dabei von Vorteil, dass durch Abrastern eines zusammenhängenden dreidimensionalen Bereiches im Gewebe ein vollständiges Volumenmodell entstehen kann und insbesondere die Lage von Grenzschichten und Einschlüssen bekannt ist. Abhängig vom konkreten Einzelfall genügt es mitunter aber auch, das Gewebe nur zweidimensional oder sogar eindimensional abzutasten, um notwendige Informationen zur Erzeugung von Zielpunkten für die Einwirkung von Behandlungs-Laserstrahlen zu gewinnen. Durch die Lageinformation der gefilterten Messpunkte liegt in der Steuereinrichtung dann ein optimales Messregime zum Fokussieren der Zielpunkte vor. Der Behandlungslaserstrahl kann dann auf einer vorteilhaften Linie zwischen den Zielpunkten in kurzer Zeit bewegt werden. Auch kann dadurch effektiv vermieden werden, Gewebe, auf das nicht eingewirkt werden soll, zu verletzen; dennoch kann eine zu behandelnde Stelle sehr dicht an sensiblen Gewebeteilen liegen, da die Messpunkte eine genaue Analyse erlauben. Hinsichtlich der Lage der Messpunkte zu den Zielpunkten kann es im Interesse einer schnellen Vermessung des Gewebes vorteilhaft sein, die Messpunkte in einem anderen Abstand zu wählen, als die Zielpunkte, beispielsweise in größeren Abstand. Zielpunkte werden dann auch zwischen Messpunkte gelegt, da es dann von Vorteil ist, mehr Zielpunkte anzufokussieren, als Messpunkte erfasst wurden.

Da die Lokalisierung des Fokusses der Beleuchtungs-Laserstrahlung in den Messpunkten über geeignete Ansteuerung der Fokussiereinrichtung (Lage des Messpunktes in der Tiefe des Gewebes) und der Ablenkeinrichtung (laterale Lage des Messpunktes) erfolgt, kann jedem Messpunkt eine eindeutige Stellung der Fokussiereinrichtung und der Ablenkeinrichtung zugeordnet werden. Die örtliche Lage des Zielpunktes ist damit ebenfalls durch geeignete Parameter von Fokussiereinrichtung und Ablenkeinrichtung definiert. Um hier eine hohe Genauigkeit zu erreichen, ist es vorteilhaft, eine Toleranzkette, die bei der Verwendung zweier getrennter Systeme mitunter unvermeidbar ist, auszuschließen, indem die Behandlungs-Laserstrahlung mittels derselben optischen Mittel im Gewebe örtlich verändert wird, mit denen auch die Lage des Fokuspunktes der Beleuchtungs-Laserstrahlung beeinflusst wird. Die damit erreichbare absolute Genauigkeit der Platzierung des Fokusses der Behandlungs-Laserstrahlung eröffnet Anwendungen, die bisher wegen der Gefahr, dicht benachbartes sensibles Gewebe ungewollt zu beeinflussen, nicht möglich waren.

Es ist deshalb bevorzugt, dass die in das Gewebe fokussierte Behandlungs-Laserstrahlung auch als Messstrahlenbündel wirkt, wobei es über dieselbe Ablenkeinrichtung und dieselbe Fokussiereinrichtung geformt und geführt wird. Die Vermessung und Diagnose sowie die Behandlung wird dann mit ein und demselben Laserstrahlbündel durchgeführt, das von derselben Laserstrahlungsquelle kommend und über dieselben optischen Mittel in das Gewebe fokussiert wird. Dadurch wird erreicht, dass erfasste Messpunkte bezüglich der optischen Durchbrüche und Sollzielpunkte auf denselben Referenzpunkt bezogen sind, wie die Zielpunkte. Für die Messung ist die Leistung der Behandlungs-Laserstrahlung reduziert, z.B. durch optische Mittel, so dass sie in ihrem Fokus laserinduzierte Signale bewirkt, die eine Messung ermöglichen, das Gewebe jedoch nicht verändern. Es ist diesbezüglich vorteilhaft, einen Energieminderer vorzusehen, der in den Strahlengang des Behandlungs-Laserstrahls geschaltet werden kann und die Energie der in das Gewebe fokussierten Behandlungs-Laserstrahlung so reduziert, dass keine irreversible Veränderung im Gewebe stattfindet, jedoch ein vom Gewebezustand oder der Gewebeart abhängiges induziertes Signal bewirkt wird, das im Detektionsstrahlengang entsprechend detektiert wird. Die vermessenen Punkte müssen dabei nicht identisch mit denjenigen Punkten sein, in denen ein optischer Durchbruch erzeugt wird, jedoch haben dann all diese Punkte einen gemeinsamen Referenzpunkt, da sie von ein und demselben Strahlengang aus ein und derselben Strahlungsquelle stammend erzeugt werden. Eine Toleranzkette, wie sie sich womöglich bei der Verwendung getrennter Systeme ergibt, ist ausgeschlossen.

Für die Messung wird die Energie der Behandlungs-Laserstrahlung, beispielsweise die Energie der einzelnen Laserpulse, mittels des Energieminderers so weit reduziert, dass im Fokuspunkt keine irreversiblen Veränderungen am Gewebe mehr hervorgerufen werden. Alternativ kann auch die Eigenschaft einer gepulsten Behandlungs-Laserstrahlquelle ausgenutzt werden, zwischen den einzelnen Laserpulsen Hintergrundstrahlung mit stark reduzierter Leistung abzugeben. Diese Hintergrundstrahlung kann für die Messung verwendet werden, und ein Energieminderer entfällt.

Die im Detektionsstrahlengang detektierten Signale sind Messpunkten zugeordnet, die jeweils durch die bestimmte Stellung des Detektionsstrahlenganges, beispielsweise durch die bestimmte Stellung einer Fokussiereinrichtung und/oder einer Ablenkeinrichtung definiert sind. Diese Signale können in einem Speicher abgelegt und in einem nachgelegten Komparator mit einem Schwellwert verglichen werden, der unveränderlich oder von der Lage der einzelnen Messpunkte abhängig gewählt sein kann. Dadurch ist es möglich, all diejenigen Messpunkte zu bestimmen, für die eine Behandlung vorzusehen ist. Die entsprechenden Lageinformationen werden an eine Steuereinrichtung weitergeleitet, die einen entsprechenden Ablauf der Erzeugung optischer Durchbrüche festlegt. Die Behandlungs-Laserstrahlung wird dann mit ihrem Fokus auf einer entsprechenden Bahn bewegt. So kann effektiv vermieden werden, Gewebe, das nicht bearbeitet werden soll, zu verletzen, auch wenn eine zu behandelnde Stelle sehr dicht an unverletzt zu bleibenden Gewebeteilen liegt. Die erzielbare Genauigkeit liegt in der Größenordnung des Fokusdurchmessers und kann, je nach Fokussierung und Strahlungswellenlänge, sogar unter 1 µm sein. Für die Erzeugung der optischen Durchbrüche wird dann der Energiereduzierung beendet.

Mit der Erfindung kann nach der Erzeugung der optischen Durchbrüche mit eingeschaltetem Energieminderer auf der bereits zur Erzeugung der optischen Durchbrüche abgefahrenen Bahn ein Messschritt durchgeführt werden, in dem die optischen Durchbrüche bzw. deren Wirkung vermessen werden. Dabei ist wiederum ein Vergleich mit den während der ersten Vermessung erfassten und im Speicher abgelegten Signalen möglich. In Abhängigkeit des Vergleichsergebnisses können einzelne oder mehrere Punkte zum Zweck einer möglichst erfolgreichen und schonenden Behandlung erneut mit Behandlungs-Laserstrahlung beaufschlagt werden, d.h. es wird ein zweiter Behandlungsschritt durchgeführt. Die Anzahl der Schritte kann somit in einer Abfolge aus Mess- und Behandlungsschritten beliebig gewählt werden. Es ist deshalb bevorzugt, dass wiederholt Messpunkte und Zielpunkte bestimmt werden, wobei an den Zielpunkten jeweils Behandlungs-Laserstrahlung appliziert wird.

Besonders bevorzugt für die Behandlungs-Laserstrahlung sind ultra-kurze Laserpulse mit Impulsdauern zwischen 1 fs und 100 ps im infraroten oder sichtbaren Spektralbereich (400 bis 1900 nm). Diese optische Strahlung kann Gewebe, insbesondere die transparenten Teile des Auges (Hornhaut, Linse, Glaskörper) durchdringen. Bei hohen Leistungsdichten, die nur im Fokuspunkt erreicht werden, lösen die Laserpulse nicht-lineare, optische Prozesse in Form von optischen Durchbrüchen aus, wie z.B. durch Mehrphotonenanregung oder Frequenzkonversion bedingt, wobei die erforderliche Leistungsdichte gewebespezifisch sein kann.

Die Erfindung ist insbesondere für das erwähnte zur Fehlsichtigkeitskorrektur vorgesehene Operationsverfahren geeignet. Daneben kann die Erfindung auch für andere ophthalmologische oder sonstige chirurgische Eingriffe verwendet werden. Dazu gehören Schnitte für die refraktive Augenchirurgie oder zur Entfernung von eingeschlossenen Fremdkörpern, Schnitte in der Hornhaut, Schnitte im Glaskörper des Auges, in der Linse oder der Sklera. Ebenso sind lokalisierte laserinduzierte Gewebeveränderungen ohne Schnittwirkungen denkbar, die Trübungen oder Verhärtungen der Hornhaut verringern. Aber auch andere Gewebe sind für Infrarotstrahlung transparent, beispielsweise die Dermis. Bei entsprechender Wahl der Wellenlänge der Behandlungs-Laserstrahlung sind auch in diesen Geweben Diagnosen und Behandlungen möglich. Auch für eine in vitro Gewebebearbeitung ist die Erfindung geeignet. So können beispielsweise in einem extrahierten Gewebestück histologische Untersuchungen durchgeführt werden. Der Behandlungs-Laserstrahl kann zur Ausführung eines histologischen Schnittes in eine gegebenenfalls zuvor vermessene Geweberegion benutzt werden. Die Schnitteigenschaften der ultrakurzen Laserpulse wirken sich dabei vorteilhaft auf die Qualität der Schnittfläche auf; kollaterale Gewebeschäden sind weitgehend ausgeschlossen.

Jeder optische Durchbruch wirkt sich auf das Gewebe aus. In den meisten Fällen bildet sich zumindest kurzzeitig ein optisches Streuzentrum, z.B. in Form einer Plasmablase.

Die aus dem Gewebe ausgehende Strahlung kann in einer besonders vorteilhaften Ausgestaltung der Erfindung rückgestreute Strahlung sein, die aus einer Beleuchtungsstrahlungsquelle stammt. Es ist deshalb eine Weiterbildung der Vorrichtung mit einer Beleuchtungsstrahlungsquelle, die Beleuchtungsstrahlung in das Gewebe einkoppelt, bevorzugt. Im erfindungsgemäßen Verfahren wird dann vorteilhafterweise Beobachtungsstrahlung in das Gewebe eingestrahlt und in Form von Rückstrahlung aus dem Gewebe ausgehende Strahlung ausgewertet. Beleuchtungsstrahlung und Behandlungsstrahlung können aus ein- und derselben Strahlungsquelle abgeleitet werden, z.B. durch Einsatz eines einschaltbaren Energieminderers.

In einer ersten Ausführungsform der Erfindung findet eine interferometrische Detektion der aus dem Gewebe ausgehenden Strahlung statt, und die Vorrichtung weist eine Beleuchtungsstrahlungsquelle auf, die zusammen mit dem Detektionsstrahlengang Teil eines Interferometeraufbaus ist. Zweckmäßigerweise wird rückgestreute Strahlung detektiert. Die Menge an rückgestreuter Strahlung trägt Informationen über Brechzahlsprünge, die an der Grenzschicht verschiedener Gewebearten (z.B. zwischen Stroma und Bowman'scher Membran einer Hornhaut) auftreten.

Ein solcher Interferometeraufbau kann beispielsweise in einer besonders zweckmäßigen Ausgestaltung als optischer Kohärenztomograph ausgebildet werden, der aus dem Gewebe ausgehende Strahlung, z.B. rückreflektierte oder gestreute Beleuchtungsstrahlung, in geeigneter Form räumlich filtert. Wird die Beleuchtungsstrahlung entlang einer optischen Achse eingestrahlt, kann die räumliche Filterung lateral, d.h. senkrecht zur optischen Achse durch eine geeignete Fokussierung der Beleuchtungsstrahlung erreicht werden. In axialer Richtung kann bei einem optischen Kohärenztomographen die Lokalisierung des Messvolumens durch eine kurze zeitliche Kohärenz der Beleuchtungsstrahlung erreicht werden. Interferenz tritt dann nur bei Rückstreuung aus dem Messvolumen auf, und das Vorliegen einer Interferenz zeigt dann, dass Strahlung aus einer bekannten Tiefe innerhalb des Gewebes rückgestreut wurde.

Es ist diesbezüglich bevorzugt, dass der Interferometeraufbau einen Messarm und einen verstellbaren Referenzarm aufweist. Die Kohärenzlänge der Beleuchtungsstrahlung bestimmt die Auflösung in axialer Richtung, da Interferenz nur auftritt, wenn Länge von Messarm und Referenzarm sich um weniger als die Kohärenzlänge der Beleuchtungsstrahlung unterscheiden.

Um ein möglichst einfach aufgebautes und kompaktes Gerät zu erreichen, ist es zu bevorzugen, dass die Behandlungs-Laserstrahlung und die Beleuchtungsstrahlung von derselben Optik auf das zu behandelnde Gewebe, beispielsweise auf die Hornhaut eines Auges, gerichtet werden. Dies wird man in der Regel dadurch erreichen, dass Beleuchtungsstrahlung und Behandlungs-Laserstrahlung über eine optische Koppeleinheit zusammengeführt werden, beispielsweise über einen Strahlteiler. Die dann von beiden Strahlen gemeinsam passierte Fokussieroptik erzeugt dann einen Fokus, der lateral und je nach Strahldivergenz vor der Zusammenführung auch axial etwa am selben Ort liegt.

Eine eigenständige laterale Verschiebung der Beleuchtungsstrahlung gegenüber der Behandlungs-Laserstrahlung kann erreicht werden, wenn vor der Zusammenführung eine verstellbare Ablenkeinrichtung, beispielsweise ein Scanner vorgesehen ist. Die Fokuslage der Beleuchtungsstrahlung kann in axialer Richtung durch Anpassung der Divergenz der Beleuchtungsstrahlung vor der Zusammenführung mit der Behandlungs-Laserstrahlung verstellt werden. Analog kann durch geeignete Vorkonditionierung des Durchmessers des Strahls der Beleuchtungsstrahlung die Fokusgröße gegenüber der Behandlungs-Laserstrahlung verändert werden.

Es ist deshalb diesbezüglich bevorzugt, dass die Beleuchtungsstrahlung in einen Lichtweg der Behandlungs-Laserstrahlung eingekoppelt ist, wobei eine verstellbare Optik verwendet ist, mit der die Divergenz der Beleuchtungsstrahlung ohne Veränderung der Divergenz der Behandlungs-Laserstrahlung veränderbar ist.

Die beim optischen Kohärenztomographen erfolgende Heterodyndetektion der rückgestreuten Strahlung verwendet vorzugsweise eine Beleuchtungsstrahlung mit möglichst großer Bandbreite, bevorzugt um eine mittlere Wellenlänge von 850 nm. Die Bandbreite ist umgekehrt proportional zur Kohärenzlänge der Beobachtungsstrahlung, die in der Heterodyndetektion die axiale Auflösung des optischen Kohärenztomographen mitbestimmt. Durch die Verwendung verschiedener Wellenlängen von Beleuchtungsstrahlung und Behandlungs-Laserstrahlung kann eine Überlagerung und Trennung der beiden Strahlengänge mit dichroitischen Strahlteilern einfach erfolgen.

Für die Realisierung der erwähnten axialen Verschiebung des Fokusses gibt es prinzipiell zwei Möglichkeiten:
a) Ist der Fokusdurchmesser der Beleuchtungsstrahlung größer als der des Behandlungs-Laserstrahls, kann ein Abtasten durch ein geeignetes Verstimmen des Interferometer, z. B. durch Verstellen des Messarmes erreicht werden. Die axiale Auflösung ist dann hauptsächlich durch die Kohärenzlänge der Beleuchtungsstrahlung bestimmt und liegt typischerweise in der Größenordnung von 10 µm. Der Verstimmbereich legt den Messbereich axial fest.
b) Ist der Fokusdurchmesser der Beleuchtungsstrahlung und der Behandlungs-Laserstrahlung in ähnlicher Größe, setzt ein axiales Abtasten eine synchrone Verstimmung des Interferometers und der Fokuslage voraus. Letzteres kann mittels der verstellbaren Optik, mit der die Divergenz der Beleuchtungsstrahlung ohne Veränderung der Divergenz der Behandlungs-Laserstrahlung veränderbar ist, vor der Zusammenführung von Beleuchtungsstrahlung und Behandlungs-Laserstrahlung erfolgen. Die axiale Auflösung der Detektion des optischen Durchbruchs ist dann sowohl von der Tiefenschärfe der durch die Heterodyndetektion inhärent konfokalen optischen Abbildung als auch von der Kohärenzlänge der Beleuchtungsstrahlung abhängig und liegt unter der erwähnten Auflösung für Fall a).

Die im Fall b) erreichte höhere Auflösung kommt natürlich auch einer lateralen Abtastung des Gebietes, in dem der optische Durchbruch erwartet bzw. erzeugt wird, zugute. Andererseits ist dann eine erhöhte Verstellgeschwindigkeit erforderlich, um ein Gebiet bestimmter Größe in gleicher Zeit abzutasten.

Eine weitere Möglichkeit zur räumlichen Selektion der aus dem Gewebe ausgehenden Strahlung, aus der das Detektionssignal gewonnen wird, ist die Verwendung einer konfokalen Abbildung. Es ist deshalb in einer zweiten Ausführungsform der Erfindung bevorzugt, dass die Detektoreinrichtung die aus dem Gewebe ausgehende Strahlung mittels einer konfokalen Abbildung erfasst. Die Vorrichtung ist dann ähnlich einem Laserscanningmikroskop, das nach dem Auflichtverfahren in Reflexion arbeitet, aufgebaut.

Das konfokale Prinzip gestattet es, ein bestimmtes Volumenelement im Gewebe auf den Detektor abzubilden und Strahlung aus dem Gewebe, die von Volumenelementen abseits des bestimmten Volumenelementes ausgeht, zu unterdrücken. Insbesondere erfolgt eine starke Unterdrückung von Strahlung aus tieferen oder höheren Ebenen. Durch die Konfokalitätsbedingung zwischen dem abgefühlten Volumenelement und der üblicherweise durch ein Pinhole-Objektiv erfolgenden Abbildung auf den Detektor ist bei entsprechender chromatischer Korrektur sichergestellt, dass Strahlung gleich welcher Wellenlänge auf dem Detektor landet, sofern sie nur aus dem ausgewählten Volumenelement stammt.

Die konfokal detektierte Strahlung ist rückgestreute Beleuchtungsstrahlung. Diese kann besonders vorteilhaft mittels eines dichroitischen Strahlteilers in den Strahlengang des Behandlungslaserstrahls eingebracht werden. Eine axiale Verschiebung des ausgewählten Volumenelementes kann dabei durch eine Fokusverstellung in der konfokalen Abbildung erfolgen, wie dies auch von Laserscanning-Mikroskopen bekannt ist. Es ist deshalb bevorzugt, dass die Detektoreinrichtung das Detektionssignal erzeugt, indem sie den Fokus der konfokalen Abbildung verstellt, vorzugsweise entlang der Strahlrichtung der Behandlungs-Laserstrahlung. Dies kann beispielsweise durch die bereits für die erste Ausführungsform erwähnte verstellbare Optik erreicht werden, die die Divergenz der Beleuchtungsstrahlung ohne Veränderung der Divergenz der Behandlungs-Laserstrahlung verändert. Mit dieser Bauweise ist ein kompakter Aufbau erreicht, da Behandlungs-Laserstrahlung und Beleuchtungsstrahlung über eine gemeinsame Optik in das Gewebe gebündelt werden.

Da die axiale Auflösung bei einer konfokalen Detektion untrennbar mit der Größe des Fokus-Spots verknüpft ist, muss man, um eine hohe Auflösung zu erreichen, die Beleuchtungsstrahlung möglichst eng im Gewebe bündeln. Für eine axiale Auflösung von ca. 10 µm ist ein Fokusdurchmesser von ca. 3 µm erforderlich. Dies kann durch geeignete Ausleuchtung des die Strahlung in das Gewebe fokussierenden Objektivs relativ unabhängig von der Fokussierung der Behandlungs-Laserstrahlung erreicht werden.

Die Abtastung in lateraler Richtung kann bei einer konfokalen Detektion auf bekannte Weise durch entsprechendes Ablenken des Beleuchtungsstrahls bzw. des Fokusses in lateraler Richtung, d.h. quer zur optischen Achse erfolgen.

In einer dritten Ausführungsform der Erfindung kann auf die axiale Verstellung des bei der konfokalen Detektion ausgewählten Volumenelements weitgehend verzichtet werden. Dazu wird das Grundprinzip der konfokalen Detektion so abgewandelt, dass nun in der konfokalen Abbildung ein dispersives Element, beispielsweise eine gezielt mit einer bestimmten Dispersion ausgestatteten Optik verwendet wird. Geht vom ausgewählten Volumenelement nun weißes Licht aus, d.h. Strahlung die aus verschiedenen Spektralanteilen, beispielsweise rotes, grünes oder blaues Licht, zusammengesetzt ist, werden bestimmte Spektralanteile nur aus einer bestimmten Tiefe der Probe durch die Optik genau in das Pinhole fokussiert und damit auf dem Detektor abgebildet. Kurzwelligere Anteile (beispielsweise blaues Licht) haben für ein bestimmtes Volumenelement dann einen Fokus auf der optischen Achse zwischen Pinhole und Pinhole-Optik. Sie divergieren damit wieder, bis sie zum Detektor gelangen, so dass nur ein kleiner Teil der Strahlung dieses Spektralanteils durch das Pinhole und zum Detektor gelangen kann. Folglich werden diese Anteile sehr effektiv unterdrückt. Gleiches gilt für langwelligere Anteile (z.B. rotes Licht), da der ihnen zugeordnete Fokus hinter dem Pinhole liegt, die Strahlen aber vorher durch das Pinhole abgeblockt werden. Nur ein bestimmter mittlerer Spektralanteil (beispielsweise grünes Licht) wird aus dem weiß abstrahlenden Volumenelement durch die Optik durch das Pinhole abgebildet.

Ein weiteres weiß abstrahlendes Volumenelement, welches sich zwischen dem gerade betrachteten Volumenelement und der Optik auf der optischen Achse befindet, kann dagegen kurzwelligere Strahlungsanteile in einen im Pinhole liegenden Fokus leiten. Entsprechendes gilt für ein Volumenelement, welches sich von der Optik aus gesehen hinter dem zuerst betrachteten Volumenelement befindet. Von dort werden nur die langwelligen Anteile genau auf das Pinhole fokussiert und können detektiert werden.

Das dispersive Element bewirkt eine Fokusverschiebung zwischen bestimmten Spektralanteilen der detektierten Strahlung, die der gewünschten axialen Auflösung entspricht. Es ist deshalb für diese Ausführungsform bevorzugt, den Detektor zur Aufnahme der Strahlung hinter dem Pinhole spektral selektiv bzw. auflösend auszubilden, beispielsweise als mehrkanaliges Spektrometer. Dabei kann das Spektrometer im einfachsten Fall nur zwei oder drei Kanäle aufweisen.

Die Kanäle des Spektrometers werden dann ausgelesen und ausgewertet, und aus der relativen Höhe der einzelnen Signale zueinander kann die Lage des die Strahlung streuenden oder emittierenden Volumenelementes auf der optischen Achse bestimmt werden.

Für die externe Beleuchtung kann beispielsweise eine Weißlicht-LED, ein Glühemitter, ein geeignet breitbandiger Laser oder eine Superlumineszenzdiode Verwendung finden.

In einer besonders einfach zu realisierenden Ausgestaltung können mehrere eigenständig ansteuerbare Strahlungsquellen mit voneinander unterschiedlich spektralen Eigenschaften verwendet werden, beispielsweise rote, grüne und blaue LED. Diese einzelnen, spektral verschiedenen Strahlungsquellen werden dann sequentiell betrieben, so dass der Detektor kein spektrales Auflösungsvermögen mehr haben muss. Die Informationen für die einzelnen Farbkanäle werden zeitsequentiell gewonnen, so dass die Auswertung wie bereits oben erwähnt verläuft. Es ist deshalb bevorzugt, dass die Beleuchtungs-Strahlenquelle mehrere, einzeln betätigbare Teilstrahlungsquellen mit unterschiedlichen spektralen Eigenschaften aufweist, so dass die spektral selektive Erfassung durch sequentielle Betätigung der Teilstrahlungsquellen bewirkbar ist. Es wird spektral unterschiedliche Beleuchtungsstrahlung sequentiell eingestrahlt und die Aufnahme der aus dem Gewebe ausgehenden Strahlung erfolgt wiederum sequentiell, so dass die entsprechende spektrale Zuordnung erreicht ist.

In den geschilderten Ausführungsformen, in denen Detektionsstrahlengang und BeleuchtungsStrahlengang im Wesentlichen auf einer gemeinsamen optischen Achse einfallen, ist zwar ein sehr kompakt bauendes Gerät erreichbar, jedoch kann mitunter eine an und für sich wünschenswerte Information über Strukturen, die auf der optischen Achse hinter einem optischen Durchbruch liegen, nur sehr schwer erhalten werden, da der optische Durchbruch für Strahlung aus dahinterliegenden Gewebeabschnitten meist eine abschattende Wirkung hat. Für Anwendungen, in denen auch eine optische Detektion in Gewebebereichen erfolgen sollen, die von der Vorrichtung aus gesehen hinter einem optischen Durchbruch liegen, ist es deshalb zweckmäßig, dass der Detektionsstrahlengang eine optische Achse aufweist, die schräg zu einer optischen Achse der Behandlungs-Laserstrahlung oder einer Beleuchtungsstrahlung liegt. Die ausgehende Strahlung wird also entlang einer optischen Achse detektiert, welche schräg zu einer optischen Achse liegt, entlang der die Behandlungs-Laserstrahlung oder eine Beobachtungsstrahlung in das Gewebe eingestrahlt wird. Dieser Ansatz erlaubt es beispielsweise die Dicke einer Hornhaut gleichzeitig mit der Erfassung des optischen Durchbruches zu bestimmen. Die Dickenbestimmung ermöglicht wiederum eine einfache Skalierung der Abmessung des optischen Durchbruches, beispielsweise eines Plasmablasendurchmessers da die Hornhautdicke ein gut bekanntes und insbesondere vor ophthalmologischen Eingriffen in der Regel exakt vermessenes Maß liefert. Damit ist auch zusätzlich eine automatische Kalibrierung zur Vermessung des optischen Durchbruches erreicht.

Die schräge Einstrahlung zur optischen Achse der Behandlungs-Laserstrahlung kommt für alle eingangs erwähnten Ausführungsformen der Erfindung in Frage. Sie bietet sich aber auch insbesondere für eine weitere, vierte Ausführungsform an, die das von Spaltlampen bekannte Prinzip weiterentwickelt. Mittels einer Spaltoptik kann eine Spaltbeleuchtung schräg zur optischen Achse eines Beobachtungsstrahlenganges eingestrahlt werden, beispielsweise in die Hornhaut. Die Schnittpunkte von Beobachtungsstrahlengang und Spaltbeleuchtung stellen einen Streulichtkanal dar, aus dem von der Spaltbeleuchtung stammende, im untersuchten Gewebe gestreute Strahlung in den Beobachtungsstrahlengang gelangen kann. Verschwenkt oder verschiebt man nun die optischen Achsen von Spaltbeleuchtung und Beobachtungsstrahlengang gegeneinander, so wandert der den Streulichtkanal bildende Schnittpunkt im untersuchten Gewebe. Beispielsweise kann eine Verschiebung von der Hornhauthinterfläche bis zur Hornhautvorderfläche vorgenommen werden.

Die Verstellbarkeit der Lage von Beobachtungsstrahlengang und Beleuchtungsstrahlengang ist mechanisch auf beliebig geeignetem Wege erreichbar. Besonders einfach ist sie auszuführen, wenn ein verstellbares Leuchtfeld eingesetzt wird, wie es aus der DE 198 12 050 A1 bekannt ist. Dann kann durch die Leuchtfeldverstellung, die beispielsweise mit einem digitalen Spiegelarray bewirkt wird, auf einfache Weise ohne aufwendige mechanische Baugruppen die gewünschte Verstellung erreicht werden.

Eine Verstellung der optischen Achsen ist entbehrlich, wenn der Beobachtungsstrahlengang ein zumindest streifen- oder linienförmiges Bild des Streulichtkanales aufnimmt. Während bei der Variante mit verstellbaren optischen Achsen keine Abbildung auf einen bildgebenden Detektor erforderlich war, ist bei fest zueinander liegenden optischen Achsen von Beleuchtungsstrahlengang und Beobachtungsstrahlengang ein Bildaufnehmer erforderlich.

In einer Variante der vierten Ausführungsform, die eine besonders universelle Messung ermöglicht, erfolgt eine scannende Ablenkung quer zur optischen Achse der Beleuchtungsstrahlung. Dies ist vorzugsweise unabhängig vom Beobachtungsstrahlengang, der einen Bildaufnehmer speist. Auch hier gewährleistet die gegenüber der Beleuchtung schräge Beobachtung, dass die erforderliche Tiefeninformation erhalten wird.

Eine solche Scanvorrichtung ist für die erfindungsgemäße Vorrichtung allgemein nützlich und immer dann vorteilhaft, wenn ein um einen zu erwartenden optischen Durchbruch liegendes Gebiet abgescannt werden soll. Es ist deshalb bevorzugt, dass eine Scanvorrichtung zum Abscannen des Gewebes verwendet wird.

Die Erfindung wird nachfolgend unter Bezugnahme auf die Zeichnung beispielshalber noch näher erläutert. In den Zeichnungen zeigen:
- Fig. 1: eine perspektivische Darstellung eines Patienten während einer laserchirurgischen Behandlung mit einem laserchirurgischen Instrument,
- Fig. 2: eine Schemadarstellung des laserchirurgischen Instrumentes der Fig. 1,
- Fig. 3: die Fokussierung eines Strahlenbündels auf das Auge des Patienten beim Instrument der Fig. 2,
- Fig. 4: einen Signalverlauf bei der Vermessung von Streuzentren, die während der laserchirurgischen Behandlung im Auge erzeugt werden,
- Fig. 5: eine schematische Darstellung zur Erläuterung einer während der laserchirurgischen Behandlung ausgeübten Schnittführung,
- Fig. 6: eine Lichtquelle für das laserchirurgische Instrument der Fig. 2,
- Fig. 7: eine Scanvorrichtung für das laserchirurgische Instrument der Fig. 1 und
- Fig. 8: eine weitere Ausführungsform einer Messvorrichtung des laserchirurgischen Instruments der Fig. 1.

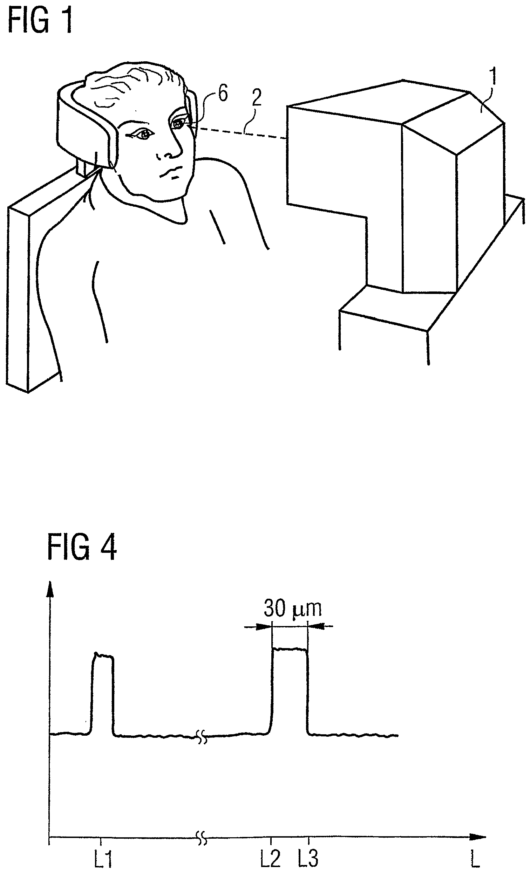

In Fig. 1 ist ein laserchirurgisches Instrument 1 dargestellt, das einen Behandlungsstrahl 2 abgibt, welcher auf das Auge 6 eines Patienten gerichtet ist. Das laserchirurgische Instrument 1 ist dabei in der Lage, einen gepulsten Behandlungsstrahl 2 zu erzeugen, so dass das in US 6.110.166 beschriebene Verfahren ausgeführt werden kann. Die Pulsdauer liegt dabei im Nano- oder Femtosekundenbereich.

Mittels des laserchirurgischen Instrumentes 1 wird eine Fehlsichtigkeit des Auges 6 des Patienten dadurch behoben, dass aus der Hornhaut des Auges 6 Material so entfernt wird, dass sich die Brechungseigenschaft der Hornhaut um ein gewünschtes Maß ändert. Das Material wird dabei dem Stroma der Hornhaut entnommen, das unterhalb von Epithel und Bowmanscher Membran und oberhalb der Decemetscher Membran und des Endothels liegt.

Die Materialentfernung erfolgt, indem durch Fokussierung des hochenergetischen gepulsten Behandlungsstrahls 2 des laserchirurgischen Instrumentes 1 im Stroma Gewebeschichten getrennt werden. Jeder einzelne optische Durchbruch initiiert dabei eine Plasmablase, so dass die Gewebeschichttrennung ein größeres Gebiet erfasst, als der Fokus des Behandlungsstrahls 2. Durch geeignete Ablenkung des Behandlungsstrahls 2 werden während der Behandlung Plasmablasen aneinandergereiht. Die aneinander liegenden Plasmablasen umschreiben dann ein Teilvolumen des Stromas: das zu entfernende Material.

Das laserchirurgische Instrument 1 wirkt durch den Behandlungsstrahl 2 wie ein chirurgisches Messer, das, ohne die Oberfläche der Hornhaut zu verletzten, direkt Material im Inneren des Stromas schneidet. Führt man den Schnitt durch weitere Erzeugung von Plasmablasen bis an die Oberfläche der Hornhaut, kann das durch die Schnittführung isolierte Material des Stromas seitlich aus der Hornhaut herausgezogen und somit entfernt werden.

Die Schnittführung des laserchirurgischen Instrumentes 1 wird gemäß zuvor ermittelten Parametern durchgeführt, damit das entfernte Teilvolumen des Stromas eine derartige Veränderung der optischen Eigenschaften der Hornhaut bewirkt, dass eine zuvor bestehende Fehlsichtigkeit weitest möglich korrigiert ist.

Es ist möglich, mittels einer automatischen Prozesskontrolle eine Regelung der Parameter des Behandlungsstrahls 2 bzw. der Ablenkung des Behandlungsstrahls 2 auf bestimmte Werte hin vorzunehmen. Das laserchirurgische Instrument 1 arbeitet dann vollautomatisch.

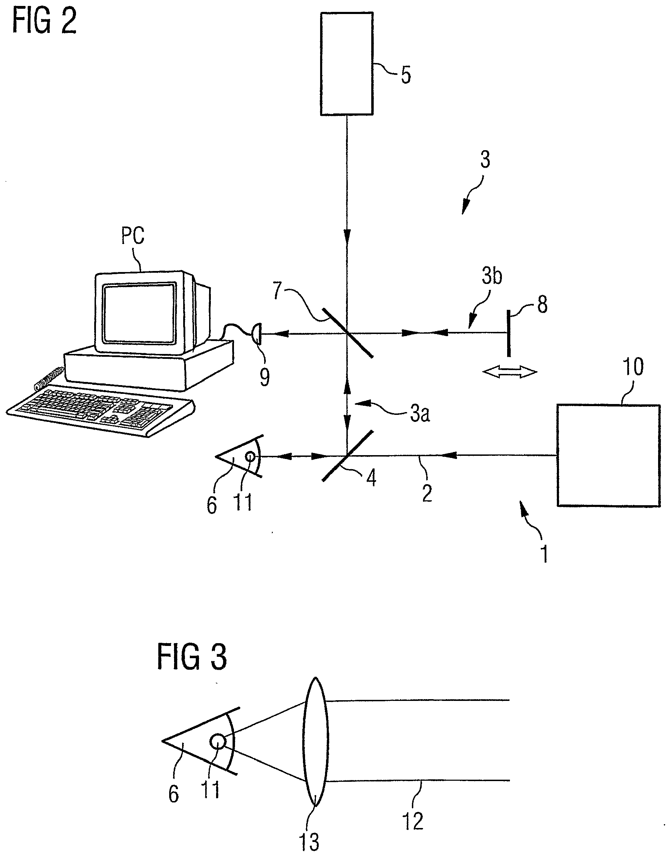

Fig. 2 zeigt eine erste Ausführungsform des laserchirurgischen Instruments 1. Eine Messvorrichtung ist hier als optischer Kohärenztomograph 3 aufgebaut und folgt dem Prinzip, wie es in der Veröffentlichung Arch. Ophtalmol., Vol. 112, S. 1584, "Micrometer-scale Resolution Imaging of the Interior Eye in vivo with Optical Coherenztomographie" von J. Izatt et al., beschrieben ist.

Der optische Kohärenztomograph 3 ist über einen Einkoppelstrahlteiler 4 in den Behandlungsstrahl 2 des laserchirurgischen Instrumentes 1 eingebunden und weist einen Messarm 3a sowie einen Referenzarm 3b auf. Der Einkoppelstrahlteiler 4 koppelt Strahlung eines Messlasers 5 in den Lichtweg des laserchirurgischen Instrumentes 1 so ein, dass die optischen Achsen von Behandlungsstrahl und Messlaserstrahl zusammenfallen. Der Fokus des Behandlungsstrahls 2 und der Fokus der Strahlung des Messlasers 5, die beide durch eine dem Einkoppelstrahlteiler 4 nachgeordnete Fokussieroptik 13 in der Hornhaut des Auges 6 gebildet werden, befinden sich lateral etwa an derselben Stelle (in Fig. 2 nicht zu sehen).

Fig. 3 zeigt wie die bereits vereinigte Strahlung des Messlasers 5 und des Behandlungsstrahls 2 als einfallender Strahl 12 mittels der Fokussieroptik 13 in die Hornhaut des Auges 6 fokussiert wird. Wenn die Energiedichte des Behandlungsstrahls 2 (Pulslänge und Strahlungsleistung) oberhalb eines bestimmten Schwellwertes liegt, tritt im Fokus ein optischer Durchbruch auf, der eine Plasmablase 11 erzeugt.

Die Strahlung des Messlasers 5 wird an der Plasmablase 11 zurückgestreut, am dichroitischen Einkoppelstrahlteiler 4 wieder ausgekoppelt und gelangt dann zu einem Messstrahlteiler 7, wo sie mit Strahlung des Messlasers 5 überlagert wird, die zuvor vom Messstrahlteiler 7 zu einem Spiegel 8 abgeteilt wurde. Die Lage des Spiegels 8 ist verstellbar. Der Messarm 3a verläuft somit vom Messstrahlteiler 7 über den Einkoppelstrahlteiler 4 zum Auge 6 und der Referenzarm 3b ist zwischen Messstrahlteiler 7 und verstellbarem Spiegel 8 gebildet.

Die aus Referenzarm 3b und Messarm 3a zum Messstrahlteiler 7 zurückkehrende Strahlung wird auf einem Photoempfänger 9 zur Interferenz gebracht. Da als Messlaser 5 eine Lichtquelle verwendet wird, die bei hoher räumlicher Kohärenz eine vergleichsweise kurze Kohärenzlänge in axialer Richtung (zeitliche Kohärenzlänge) von etwa 10 µm aufweist, erscheint an der Photodiode 9 nur dann ein Interferenzmuster, wenn die optischen Längen von Messarm 3a und Referenzarm 3b maximal um die zeitliche Kohärenzlänge der Strahlung des Messlasers 5 differieren. Um mit dem optischen Kohärenztomographen 3 eine möglichst hohe Auflösung zu erreichen, wird ein Messlaser 5 mit einer möglichst kurzen zeitlichen Kohärenzlänge verwendet, im Ausführungsbeispiel liegt diese unter 10 µm.

Um eine Beeinflussung des optischen Kohärenztomographen durch den Behandlungsstrahl 2 des laserchirurgischen Instruments 1 zu verhindern, der von einem Behandlungslaser 10 abgegeben und auf das Auge 6 gelenkt wird, ist der Wellenlängenbereich der Strahlung des Messlasers 5 vom Wellenlängenbereich der Beobachtungsstrahlung 2 verschieden und der Einkoppelstrahlteiler 4 hat geeignete dichroitische Eigenschaften. Beispielsweise wird ein Messlaser 5 verwendet, der Strahlung bei etwa 850 nm mit einer möglichst großen Bandbreite abgibt. Eine große Bandbreite fördert die axiale Auflösung, da die Bandbreite umgekehrt proportional zur zeitlichen Kohärenzlänge des Lichtes ist. Bei Verwendung verschiedener Wellenlängen für die Strahlung des Messlasers 5 und Beobachtungsstrahl 2 kann eine Überlagerung und Trennung der Strahlen mit einem dichroitischen Einkoppelstrahlteiler 4 erfolgen. Zusätzlich oder alternativ können im Strahlengang des optischen Kohärenztomographen geeignete Filter eingesetzt werden.

Zur Messung des Ortes einer Plasmablase 11 wird der Spiegel 8 verschoben, bis an der Photodiode 9 ein Interferenzmuster auftritt. Zeigt das Signal der Photodiode 9, das von einem Computer PC ausgelesen wird, eine Interferenz der Strahlung aus Messarm 3a und Referenzarm 3b an, so sind Messarm 3a und Referenzarm 3b gleich lang und der Abstand zum Fokus der Strahlung des Beobachtungslasers 5 in der Hornhaut des Auges 11 ist bekannt, wobei die Messungenauigkeit die zeitliche Kohärenzlänge der Strahlung des Messlasers 5 ist, d.h. die Kohärenzlänge in Strahlrichtung.

Die Rückstreuung von Strahlung des Messlasers 5 am Auge 6 gibt eine Aussage über die Brechzahlsprünge in der Hornhaut des Auges 6, die an der Grenzschicht verschiedener Gewebe, z.B. zwischen Stroma und Bowmanscher Membran, als auch am Ort der Wechselwirkung von Behandlungsstrahl 2 und Hornhaut des Auges 6 sowohl unterhalb als auch oberhalb einer Schwelle auftreten, bei der es zum erwähnten optischen Durchbruch mit Photodisruption und/oder -ablation kommt.

Damit ein möglichst großer axialer Bereich vermessen werden kann, ist zusätzlich zu einem großen Verstellweg für den Spiegel 8 zwischen den Messstrahlteiler 7 und den Einkoppelstrahlteiler 4 eine Einrichtung zur Veränderung der Divergenz der Strahlung des Messlasers 5 geschaltet (in Fig. 2 zur Vereinfachung nicht gezeigt), so dass vor der Einkopplung in den Behandlungsstrahl 2 die Divergenz der Strahlung des Messlasers 5 verstellt werden kann. Durch die dem Einkoppelstrahlteiler 4 nachgeschaltete Fokussieroptik 13 erfolgt dann die Fokussierung von Messstrahl 2 und Strahlung des Messlasers 5 in unterschiedliche Foki, wobei durch die Verstellung der die Divergenz der Strahlung des Messlasers 5 beeinflussenden Einrichtung die Fokuslage der Strahlung des Messlasers 5 unabhängig vom Fokus des Behandlungsstrahls 2 verstellt werden kann. Der Fokus der Beobachtungsstrahlung ist somit entlang der optischen Achse gegenüber dem Fokus der Beobachtungsstrahlung 2 verstellbar. Damit wird rückgestreute Strahlung auch entlang der optischen Achse des Behandlungsstrahls 2 in einem Bereich erfassbar, der größer als der Verstellweg des Spiegels 8 ist.

Die Einrichtung zur Verstellung der Divergenz des Messlasers 5 erlaubt es weiter, die Spotgröße der fokussierten Strahlung des Messlasers 5 in etwa so groß zu gestalten, wie die Spotgröße des fokussierten Beobachtungsstrahls, da ein Scannen in axialer Richtung, d.h. entlang der Achse des Beobachtungsstrahls 2, dann über eine synchrone Verstellung der divergenzverändernden Einrichtung und des Spiegels 8 gelingt. Durch die bei dieser Variante relativ höhere Fokussierung der Strahlung des Messlasers 5 liefert der Kohärenztomograph 3 damit eine axiale Auflösung, die sogar besser ist, als die zeitliche Kohärenzlänge der Strahlung des Messlasers 5. Die Messgenauigkeit steigt folglich. Ist dies nicht erforderlich, kann auf die die Divergenz beeinflussende Vorrichtung zwischen dem Einkoppelstrahlteiler 4 und dem Messstrahlteiler 7 verzichtet werden. Eine axiale Verschiebung des Bereiches, von dem rückgestreute Strahlung zu Interferenz am Photoempfänger führen kann, erfolgt dann ausschließlich durch Verstellung des Spiegels 8, und die axiale Auflösung wird hauptsächlich durch die zeitliche Kohärenzlänge des Messlasers 5 bestimmt (typischerweise in der Größenordnung von 10 µm).

Fig. 4 zeigt ein Signal, wie es mit einem laserchirurgischen Instrument 1 mit einem optischen Kohärenztomographen 3 in der Bauweise der Fig. 2 erhalten wird. Hierbei ist das Signal als Funktion der Verstellung des Spiegels 8 aufgetragen, wobei in Fig. 4 der Verstellweg L des Spiegels 8 als Variable eingezeichnet ist. Das Signal ist aus der mit dem Photoempfänger 9 detektierten Interferenzerscheinung abgeleitet und wurde vom Computer PC erzeugt. Es codiert die Modulationstiefe der Interferenz und weist immer dann einen hohen Pegel auf, wenn das Signal des Photoempfängers 9 eine Interferenzerscheinung anzeigt.

Wie Fig. 4 zeigt, tritt eine erste Interferenz bei einer Verschiebung L1 auf. Diese Interferenz ist durch am Epithel der Hornhaut rückgestreute Strahlung verursacht. Mit weiterer Verschiebung des Spiegels 8 tritt ein zweiter Peak des Signals bei einer Verschiebung L2 auf. Diese Interferenz ist durch den Brechzahlsprung an der Plasmablase 11 verursacht. Die Interferenz hält bis zu einer Verschiebung L3 an. Die Größe der Plasmablase wirkt sich zwar auf den Abstand L2, L3 aus, jedoch erlaubt es in der realisierten Ausführungsform die Messauflösung des Kohärenztomographen 3 nicht, den bei etwa 30 µm oder darunter liegenden Blasendurchmesser zu vermessen. Bei Verwendung einer Strahlungsquelle mit kürzerer zeitlicher Kohärenzlänge ist auch eine solche Durchmesservermessung möglich.

Der Abstand zwischen der dem Epithel zugeordneten Verschiebung L1 und dem Beginn des Interferenzpeaks bei der Verschiebung L2 gibt an, wie tief die Plasmablase unter dem Epithel, d.h. der Hornhautoberfläche liegt. Aus dem Signal kann so die Lage der Plasmablase 11, die vom Behandlungsstrahl 2 in der Hornhaut des Auges 6 verursacht wurde, in Form des Abstandes vom Epithel erfasst werden. Alternativ zum Bezug auf das Epithel kann natürlich eine Referenzierung auf das Endothel erfolgen. Hier ist in der Darstellung der Fig. 4 bei einer weitergehenden Verschiebung ein erneuter Peak durch am Endothel rückgestreute Strahlung zu erwarten. Anstelle des Endothels bzw. des Epithels kann natürlich auch ein Brechzahlsprung an der Bowmanschen oder Decementschen Membran als Bezug verwendet werden.

Der Computer PC, der das Signal des optischen Kohärenztomographen 3 ausliest, generiert das Signal. Er dient weiter zur Steuerung des Behandlungslasers 10, wozu er zum einen eine Anzeige speist, die die Lage der vom Behandlungsstrahl 2 erzeugten Plasmablase 11 angibt.

Zum anderen wird diese axiale Lage der Plasmablase zur Steuerung des Behandlungsstrahls 2 ausgewertet und verwendet, um zu garantieren, dass der Schritt zur Isolierung des Teilvolumens im Stroma wie gewünscht ausgeführt wird.

In einer vollautomatisch die Einhaltung von behandelnden Chirurgen vorgegebener Werte überwachenden Ausführung regelt der Computer Parameter des Behandlungsstrahls 2. Das chirurgische Instrument arbeitet dann mit einer Online-Kontrolle und -Regelung.

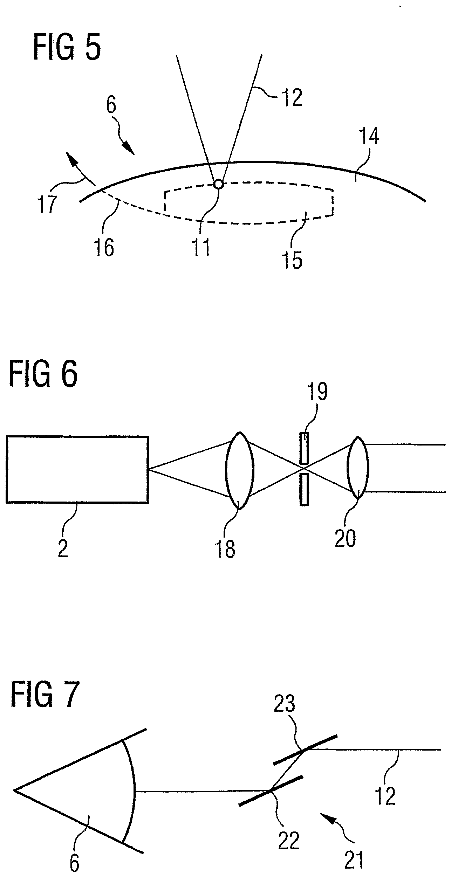

Der Messlaser 5 ist wie in Fig. 6 gezeigt aufgebaut. Er besteht aus einem Laser 2 sowie einer nachgeordneten strahlformenden Einheit aus einer Linse 18, einer Blende 19 und einer weiteren Linse 20. Der so erzeugte Strahldurchmesser ist an den Strahldurchmesser des Behandlungsstrahls 2 angepasst. Durch Verstellung der Linsen 18 und 20 kann zur Auflösungsoder Messbereichsverstellung der Strahldurchmesser zusätzlich verkleinert oder vergrößert werden. Zudem kann durch Verstellung der Linse 20 die Divergenz verändert werden und damit die Fokuslage des Messstrahles eingestellt werden.

Fig. 5 zeigt in einer schematischen Darstellung den unter Online-Kontrolle vom chirurgischen Instrument 1 ausgeführten Schnitt. Der einfallende Strahl 12 wird von der Fokussieroptik 13 in die Hornhaut 14 des Auges 6 fokussiert. Die Plasmablase 11, die mit einem Puls des Behandlungsstrahls 2 erzeugt wird, wirkt, wie bereits ausgeführt, als chirurgisches Messer, das im Inneren der Hornhaut 14 Gewebeschichten trennt. Durch Verstellung des einfallenden Strahls 12 mittels einer in Fig. 7 dargestellten Scaneinrichtung 21 aufweisend um orthogonale Achsen drehbare Scannerspiegel 22, 23 wird eine Vielzahl von Plasmablasen nebeneinander gesetzt und ein Lenslett 15 umschrieben, das aus dem Stroma der Hornhaut 14 herausgeschnitten wird. Eine mögliche Schnittform, dies zu erreichen, ist in der US 6.110.166 dargestellt, die eine spiralförmige Aneinanderreihung von Plasmablasen 14 beschreibt.

Nach dieser Schnittführung um das Lenslett 15 herum wird ein seitlicher Schnitt 16 vorgenommen, der es erlaubt, das nunmehr isolierte Lenslett 15 in Richtung 17 aus der Hornhaut 14 zu entnehmen. Die Schnittführung hat dabei den Vorteil, dass im zentralen Sichtbereich, in dem die optische Korrektur durch Entnahme des Lensletts 15 bewirkt wird, kein durch das Endothel führender Schnitt nach außen vorliegt. Stattdessen liegt der seitliche Schnitt 16 am optisch weniger bedeutsamen Rand der Hornhaut 14.

Zum Verstellen des einfallenden Strahles 12 dient die Scanneinrichtung 21, die in Fig. 7 dargestellt ist. Sie weist zwei Scannerspiegel 22, 23 auf, die unabhängig voneinander um zwei senkrecht zueinander liegende Achsen drehbar sind. Damit kann der einfallende Strahl 12 zweidimensional über die Hornhaut 14 geführt werden. Eine axiale Verstellung des einfallenden Strahls 12 erfolgt durch Verstellung der Fokussieroptik 13, d.h. durch Veränderung der Lage des Fokuspunktes, in dem die für einen optischen Durchbruch ausreichende Energiedichte und somit die Plasmablase 11 entsteht.

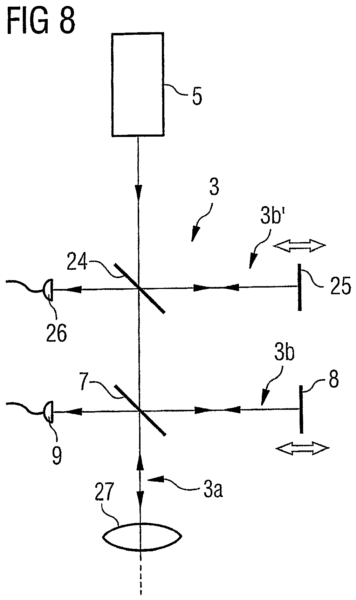

Um die axiale Lage der erzeugten Plasmablase genau zu bestimmen, wird, wie anhand des in Fig. 4 dargestellten Signals geschildert, der Spiegel 8 des optischen Kohärenztomographen 3 verstellt. Dies kann beispielsweise durch eine Oszillation erfolgen, die jedes Mal den Bereich zwischen den Stellungen L1 und L2 durchläuft. Im Falle einer axial hochauflösenden Messeinrichtung kann eine solche große Verstellung des Spiegels 8 jedoch mitunter mechanisch sehr aufwendig bzw. zeitraubend sein. Für solche Fälle ist die in Fig. 8 schematisch dargestellte Bauweise des optischen Kohärenztomographen 3 vorgesehen, die mehrere, z. B. zwei Referenzarme 3b und 3b' aufweist, welche sich einander im Wesentlichen gleichen, d.h. jeweils einen verstellbaren Spiegel 8 (Referenzarm 3b) bzw. 25 (Referenzarm 3b') haben. Jedem Referenzarm 3b, 3b' ist ein Photoempfänger 9, 26 zugeordnet.

Die Länge des Referenzarmes 3b kann unabhängig von der Länge des Referenzarmes 3b' eingestellt werden. Somit kann gleichzeitig mit hoher Auflösung an der Plasmablase und am Epithel rückgestreute Strahlung, indem z.B. der Referenzarm 3b' auf den Reflex zu einer Referenzfläche, d.h. dem Epithel eingestellt wird, und der Referenzarm 3b die Plasmablasengrenzfläche erfasst.

Fig. 8 zeigt darüber hinaus eine mögliche Bauweise der bereits bezüglich Fig. 2 angesprochenen aber dort nicht eingezeichneten Einrichtung zur Veränderung der Divergenz der Strahlung des Messlasers 5. Beim Kohärenztomographen der Fig. 8 handelt es sich um eine verstellbare Zoom-Optik 27.

Der optische Kohärenztomograph 3, wie er in den Bauweisen gemäß Fig. 2 oder 8 zur Anwendung kommt, bewirkt eine räumliche Selektion des Gebietes, aus dem rückgestreute Strahlung aufgenommen wird. Eine axiale Selektion erfolgt durch die Interferometerkonstruktion mit dem Messarm 3a und dem Referenzarm 3b (bzw. weiteren Paaren davon). Die Fokussieroptik 13 bewirkt eine laterale Selektion durch die Fokussierung. Die räumliche Selektion ist frei wählbar, d.h. unabhängig von der Lage des Behandlungsstrahlfokus, wenn der optische Kohärenztomograph mit einer eigenständigen Scaneinrichtung ähnlich der Fig. 7 ausgestattet ist, da dann mit hoher Auflösung das Gebiet um einen optischen Durchbruch bzw. eine Plasmablase 11 abgescannt werden kann. Mit der Zoom-Optik 27 kann das Gebiet der Plasmablase 11 nicht nur lateral, d.h. quer zur optischen Achse des Behandlungsstrahls 2, sondern auch axial entlang dieser optischen Achse in einem großen Messbereich angefühlt werden.

Einer zweiten Ausführungsform einer laserchirurgischen Instrumentes 1 liegt das Prinzip zugrunde, durch geeignete räumliche Filterung am Auge 6 und dabei insbesondere an einer dort erzeugten Plasmablase 11 rückgestrahlte Strahlung mit hoher und verstellbarer Tiefenschärfe so zu erfassen, dass zumindest die axiale Ausdehnung einer streuenden Struktur in der Hornhaut des Auges, beispielsweise der durch den optischen Durchbruch erzeugten Plasmablase 11, gemessen werden kann. Dafür ist ein konfokales Mikroskop vorgesehen, das nach Art eines Laserscanning-Mikroskopes arbeitet.

Das konfokale Mikroskop verfügt über einen Laser, dessen Strahlung von einer optischen Anordnung hinsichtlich der Strahlparameter wie Taillienlage und Strahlquerschnitt angepasst wird. Über einen Teiler wird die so erhaltene Beleuchtungsstrahlung zu einer Scaneinrichtung geleitet, die wie die Scaneinrichtung der vorherigen Bauweisen zwei Scannerspiegel aufweist. Die Scannerspiegel sind eng benachbart und in unmittelbarer Nähe einer Pupille des Strahlengangs angeordnet. Sie haben senkrecht aufeinander stehende Drehachsen und können separat angesteuert werden. Eine Scanoptik sammelt für die von der Betätigung der Scaneinrichtung abhängigen Strahlauslenkung die Strahlung in einer Aperturebene, von wo ein Objektiv 36 ein Spotbild erzeugt, das in einer im Auge 6 gelegenen Objektebene liegt. Im Bereich dieser Objektebene wirkt auch der Behandlungsstrahl 2. Der Behandlungsstrahl 2 stammt wie bei den vorherigen Ausführungsformen aus einem Behandlungslaser 10 und wird über einen Einkoppelstrahlteiler 4 mit dem Strahlengang des konfokalen Mikroskops vereinigt.

In der Objektebene z.B. an einer Plasmablase 11 rückgestreute Strahlung läuft den geschilderten optischen Weg bis zum Strahlteiler zurück. Die vom abgefühlten Volumenelement aus der Objektebene stammende, d.h. am Auge 6 rückgestreute Strahlung wird nach Durchlaufen des Strahlteilers in einem Detektionsarm detektiert. Je nach Ausgestaltung des Strahlteilers wird die Strahlung zumindest teilweise, bei dichroitischer Ausgestaltung des Strahlteilers auch nahezu vollständig zu einem Interferenzfilter und einer Linse geleitet, die von dem rückstreuenden Objekt in der Objektebene ein Spotbild in einer Pinholeebene erzeugt, in der eine Pinholeblende liegt. Der Pinholeblende ist ein Photomultiplier nachgeschaltet, der ein Signal liefert, das von einer angeschlossenen Auswerteeinheit unter Rückgriff auf die aktuelle Stellung der Scaneinrichtung in ein Bildsignal umgewandelt wird.

Mit der Größe der Pinholeblende kann die Größe der in der Objektebene zu detektierenden Objektstruktur eingestellt werden. Noch viel entscheidender ist es jedoch, dass mit kleiner werdendem Durchmesser der Pinholeblende eine gesteigerte Tiefendiskrimination in der Objektebene einhergeht, d.h. die Größe der Pinholeblende gibt vor, aus welchem axialen Bereich um die Objektebene herum Strahlung zum Photomultiplier gelangen kann.

Das im konfokalen Mikroskop verwendete konfokale Verfahren gestattet es, bestimmte Strahlung aus einem Volumenelement im untersuchten Objekt auf den Photomultiplier zu lenken und Strahlung, die aus anderen Volumenelementen emittiert wird, so gut wie vollständig zu unterdrücken. Insbesondere wird Strahlung aus tiefer oder höher gelegenen Gewebeschichten ausgeblendet.

Die Pinholeblende und die Objektebene, aus der Strahlung detektiert wird, liegen in konjugierten Ebenen. Durch eine Verstellung dieser Ebenen, beispielsweise durch Verstellung der Linse, ist es möglich, einen axialen Tiefenscan derart durchzuführen, dass die Objektebene, aus der die Strahlung zum Photomultiplier geleitet wird, verstellt wird. Diese Verstellung ist erforderlich, um beispielsweise eine Plasmablase 11 in axialer Richtung, d.h. entlang der Achse, auf der der Behandlungsstrahl 2 einfällt, zu vermessen.

Eine gesteigerte Unabhängigkeit zwischen axialer Lage der erwähnten Objektebene und dem Fokuspunkt des Behandlungsstrahls 2 kann dadurch erreicht werden, dass die bezüglich der Bauweisen gemäß Fig. 2 und 8 bereits erwähnte divergenzverändernde Einrichtung, beispielsweise in Form einer Zoom-Optik, der Einkopplung des Behandlungsstrahls 2 über den Einkoppelstrahlteiler 4 vorgeordnet wird. Durch geeignete Verstellung dieser Einrichtung wird die Objektebene nahezu beliebig gegenüber der Fokusebene des Behandlungsstrahls 2 verstellt.

Bei der geschilderten Bauweise ist zur baulichen Vereinfachung eine einzige Scaneinrichtung vorgesehen, die synchron sowohl die laterale Lage des Spotbildes, von dem reflektierte Strahlung zum Photomultiplier geleitet wird, als auch des Fokuspunktes des Behandlungsstrahls 2 verändert. Laterale Verschiebungen des Spots innerhalb der Objektebene gegenüber dem Fokuspunkt des Behandlungsstrahls 2 können nicht eingestellt werden.

Bei einem Einsatz zweier unabhängiger Scaneinrichtungen ist diese Beschränkung aufgehoben. Dann kann der Beleuchtungs- und Detektionsstrahlengang des konfokalen Mikroskops unabhängig gegenüber dem Behandlungsstrahl 2 am Auge 6 verstellt werden. Der Einkoppelstrahlteiler ist dann durch einen Einkoppelstrahlteiler ersetzt, der in Beleuchtungsrichtung der Scaneinrichtung des konfokalen Mikroskops nachgeordnet ist. Der Behandlungslaser 10 verfügt hier über einen eigenen Behandlungsscanner, der unabhängig von der Scaneinrichtung des konfokalen Mikroskops betätigbar ist. Diese aufwendigere Bauweise gestattet es, dass das konfokale Mikroskop aus dem Auge 6 Strahlung an Punkten aufnehmen kann, die völlig unabhängig von der Fokuslage des Behandlungsstrahls 2 und mithin von der Erzeugung von Plasmablasen 11 gewählt werden können. Dann kann nicht nur die axiale Ausdehnung einer Plasmablase 11 ermittelt werden, sondern es sind auch laterale Abmessungen erfassbar.

In der geschilderten Bauweise für die Messvorrichtung des laserchirurgischen Instrumentes 1 wird über den Laser, die optische Anordnung sowie den Einkoppelspiegel eine separate Beobachtungsstrahlung erzeugt. Je nach Anwendung kann darauf verzichtet werden und lediglich am Auge 6 eine konfokale Detektion von Streulicht vorgenommen werden, das vom Behandlungsstrahl 2 herrührt. Zusätzlich kann auch eine phasenempfindliche Detektion, z.B. nach dem Nomarski-Verfahren angewendet werden, wie es in der Veröffentlichung Padawer J., "The Nomarski interference-contrast microscope. An experimental basis for image interpretation." J. Royal Microscopical Society (1967), 88, S. 305-349, deren Offenbarungsgehalt hier expliziert einbezogen wird, beschrieben ist.

Bei der geschilderten Bauweise wird eine besonders gute Empfindlichkeit erreicht, wenn der Einkoppelstrahlteiler dichroitisch ausgebildet ist, d.h. eine strahlumlenkende Wirkung im Wesentlichen nur für die Wellenlängen des Behandlungsstrahls 2 hat. Diese dichroitische Eigenschaft ist auch für die Variante ohne separate Beleuchtungsstrahlung vorteilhaft, da sich gezeigt hat, dass in Plasmablasen 11 breitbandig Strahlung auch außerhalb des Spektralbereichs des Behandlungsstrahls 2 entsteht.

Das konfokale Detektionsverfahren wird üblicherweise mit einer Optik ausgeführt, die möglichst gut chromatisch korrigiert ist. Dadurch ist sichergestellt, dass die Strahlung aus einem bestimmten Volumenelement wellenlängenunabhängig am Detektor, beispielsweise dem Photomultiplier landet.

In einer die zweite Ausführungsform abwandelnden dritten Ausführungsform wird die Optik, die das Licht vom Objektiv auf die Pinholeblende 40 abbildet, dagegen bewusst dispersiv ausgelegt. Dies betrifft den Detektionsarm des konfokalen Mikroskops.

Ein einfallendes Detektionsstrahlenbündel wird von der nun mehrteilig ausgebildeten Linse in ein fokussiertes Detektionsstrahlenbündel umgesetzt. Aufgrund der dispersiven, d.h. wellenlängenabhängigen Fokussierungseigenschaften der die Linse bildenden Optikgruppe ist die effektive Brennweite für Licht im blauen Spektralbereich deutlich kürzer als für Licht im roten Spektralbereich. Damit wird von einem Volumenelement ausgehendes weißes Licht, das aus roten, grünen und blauen Spektralanteilen zusammengesetzt ist, in unterschiedliche Foki fokussiert. Die blaue Strahlung wird in eine Fokusebene gebündelt, die vor einer Fokusebene für grüne Strahlung liegt, der wiederum eine Fokusebene für rote Strahlung nachgeordnet ist. Die dispersiven Eigenschaften der Linse werden zur spektralen Selektion bzw. zum Gewinnen einer weiteren Tiefeninformation ausgenutzt. Dabei fokussiert die dispersive Optik der Linse für ein gegebenes Volumenelement nur grüne Spektralanteile genau in der Pinholeblende, da nur für diese Spektralanteile die Fokusebene in der Ebene der Pinholeblende liegt. Der Fokus der blauen Strahlung liegt auf der optischen Achse vor der Ebene der Pinholeblende, so dass die blaue Strahlung auf dem Weg zwischen ihrer Fokusebene und der Ebene der Pinholeblende wieder divergiert. Dadurch wird nur ein kleiner, nahezu verschwindender Teil an blauer Strahlung durch die Pinholeblende transmittiert und es ergibt sich eine effektive Unterdrückung der aus dem gegebenen Volumenelement stammenden blauen Strahlung. Dies gilt gleichermaßen für die rote Strahlung, da deren Fokusebene hinter der Ebene der Pinholeblende liegt; die rote Strahlung aus dem Volumenelement wird also durch die Pinholeblende ebenfalls abgeblockt. Effektiv gelangt somit aus dem Volumenelement nur grüne Strahlung zum Detektor, der hier als Spektrometer ausgebildet ist.

Betrachtet man nun ein weiteres weiß abstrahlendes Volumenelement, das sich auf der optischen Achse zwischen dem zuvor betrachteten Volumenelement und der Optik befindet, so wird dessen Grünanteil nun in einer Ebene hinter der Ebene der Pinholeblende 40 fokussiert. Die blaue Strahlung aus diesem weiteren Volumenelement hat allerdings einen Fokus in der Pinholeblende, wenn der axiale Abstand der beiden Volumenelemente genau der Fokusverschiebung entspricht, wobei hier noch die Vergrößerung der optischen Abbildung zu berücksichtigen ist. Analoges gilt für ein drittes Volumenelement, welches sich auf der optischen Achse vom Objektiv aus gesehen entsprechend hinter dem zuerst erläuterten Volumenelement befindet. Von diesem dritten Volumenelement können nur rote Strahlungsanteile die Pinholeblende passieren. Durch die Pinholeblende gelangt somit ein spektrales Strahlungsgemisch, wobei die Spektralinformation die axiale Lage des die Strahlung abgebenden Volumenelementes kodiert.

Die spektrale Analyse der durch die Pinholeblende fallenden Strahlung mittels des Spektrometers ermöglicht deshalb eine axiale Auflösung, ohne das konfokale Mikroskop verstellen zu müssen, wobei die spektrale Information eine Tiefeninformation über das Volumenelement trägt, aus dem die Strahlung des Spektralbereiches stammt. Aus der relativen Höhe einzelner Spektralkanäle wird die axiale Lage des Volumenelementes, aus dem die Strahlung des Spektralkanales stammt, bestimmt werden. Im einfachsten Fall genügt dabei eine Auswertung mit zwei oder drei Kanälen.

Weiter wird auf diese Weise die Größe eines strahlenden Volumenelementes bestimmt. Liegt bei einer dreikanaligen Spektralanalyse ein kleines strahlendes Volumenelement zugrunde, so registriert jeweils nur ein Kanal ein über einem bestimmten Schwellwert liegendes Signal. Im Übergangsbereich können auch zwei Signale ein entsprechendes Signal zeigen, niemals jedoch alle drei Kanäle, so lange das Volumenelement kleiner ist, als die Fokusablage zwischen den spektral am weitesten auseinanderliegenden Kanälen. Liegt dagegen ein großes strahlendes Volumenelement vor, wird das Spektrometer im Wesentlichen in allen Kanälen etwa gleiche Signale liefern. Die Breite der spektralen Verteilung, die vom Spektrometer angezeigt wird, trägt also Informationen über die Größe des Volumenelementes. Je breiter die spektrale Verteilung ist, desto größer ist das Volumenelement.

Die durch die dispersive Optikgruppe der Linse erreichte Fokusablage dF als Funktion der Wellenlänge LAMBDA ergibt für einen Spektralbereich von blau bis rot eine Fokusverschiebung in der Größenordnung von mindestens 1 mm. Mit dem geschilderten spektralselektiv detektierenden konfokalen Mikroskop können also Plasmablasen bis zu 1 mm maximaler Ausdehnung erfasst werden. Da dieser Messbereich in der Regel ausreicht, kann die dritte Ausführungsform auf eine axiale Verstellung der Objektebene des konfokalen Mikroskops verzichten und dennoch für eine Online-Kontrolle ausreichende Information über die axiale Ausdehnung einer Plasmablase 11 bereitstellen.

Natürlich spielt dabei die Spektralverteilung der aus dem untersuchten Objekt stammenden Strahlung eine Rolle. Die spektrale Detektion konfokal aufgenommener Strahlung bei Verwendung einer dispersiven Optik erfordert, dass aus der Objektebene Strahlung mit einer gewissen breitbandigen Spektralverteilung verfügbar ist. Da die spektrale Verteilung der Abstrahlung einer Plasmablase 11 nicht ausreichend zeitlich oder spektral gleichbleibende Eigenschaften aufweist, ist eine externe Beleuchtung vorgesehen. Die externe Beleuchtung muss die erforderliche spektrale Bandbreite aufweisen, beispielsweise kann eine Weißlicht-LED oder eine Glühlampe verwendet werden.

In einer Variante der dritten Ausführungsform wird kein Spektrometer eingesetzt. Statt dessen erfolgt eine Beleuchtung des zu detektierenden Volumenelementes bzw. Spots mit spektral gesteuerter Strahlung, wobei die Strahlung sequentiell spektral verstellt wird. In einer einfachen Bauweise werden zur Beleuchtung eine blaue, eine grüne und eine rote LED verwendet, die sequentiell betätigt werden. Die Information über die einzelnen Spektralkanäle wird dann zeitsequentiell erfasst, so dass durch die geeignete Gestaltung der Beleuchtungsquelle in dieser Abwandlung mit einem kostengünstigen Detektor gearbeitet werden kann, der keine oder nur ansonsten unzureichende spektrale Diskriminierungsfähigkeit zeigt.

Eine vierte Ausführungsform eines laserchirurgischen Instrumentes 1 macht sich für die Messvorrichtung das Prinzip einer Spaltlampe zunutze, indem eine Beleuchtungsstrahlung auf einer optischen Achse schräg zu einer optischen Achse, unter der eine Beobachtung erfolgt, eingestrahlt wird.

Die Messvorrichtung ist als Spaltlampenanordnung aufgebaut, die mittels einer Spaltoptik die Hornhaut 14 des Patientenauges 6 spaltförmig beleuchtet. Die Spaltoptik bildet dabei entlang einer optischen Achse der Spaltbeleuchtung in die Hornhaut ein sehr schmales Lichtbündel ab. Dieses Lichtbündel kann beispielsweise mit einer Spaltblende oder einem als steuer- oder programmierbare Blende dienenden Bauteil, wie einem Mikrospiegelarray o.ä., erzeugt werden. In dieser Bauweise wird ein Mikrospiegelarray verwendet, das mit Strahlung beleuchtet wird, wobei nur eine oder wenige Spiegelzeilen das Licht zur Hornhaut 14 reflektieren. Die Spaltbeleuchtung über die Spaltoptik kann bezüglich des Einstrahlwinkels, d.h. bezüglich der Lage der optischen Achse, in einer Scanrichtung verstellt werden. Dies erfolgt durch geeignete Ansteuerung des Mikrospiegelarrays.

In der Hornhaut wird die eingebrachte spaltförmige Beleuchtung an Streuzentren, beispielsweise dem Epithel, dem Endothel oder durch das laserchirurgische Instrument 1 erzeugten Plasmablasen 11 gestreut. Das Streulicht wird von einem Objektiv aufgenommen, das auch den Behandlungsstrahl in die Hornhaut 14 fokussiert. Der Behandlungsstrahl des laserchirurgischen Instrumentes 1 ist auch in dieser Ausführungsform über den Einkoppelstrahlteiler 4 in den Strahlengang eingekoppelt. Der Einkoppelstrahlteiler 4 trennt das vom Objektiv aufgenommene Streulicht ab, und leitet es mittels einer Abbildungsoptik auf einen Photoempfänger. Objektiv, Einkoppelstrahlteiler 4 und Abbildungsoptik bilden einen Beobachtungsstrahlengang.

Der Photoempfänger nimmt nur Strahlung auf, die aus dem Kreuzungspunkt der optischen Achse der Spaltbeleuchtung und der optischen Achse des Selektions- oder Beobachtungsstrahlenganges stammt. Am Kreuzungspunkt ist ein Streulichtkanal gebildet, aus dem durch die Spaltbeleuchtung bewirktes Streulicht aufgenommen wird.

Durch die scannende Bewegung der Spaltbeleuchtung wird der Streulichtkanal entlang der optischen Achse des Detektionsstrahlenganges verschoben, wobei eine Verschwenkung der optischen Achse der Spaltbeleuchtung nach links z. B. den Streulichtkanal Richtung Endothel, eine Verschwenkung nach rechts in Richtung Epithel verschiebt. Die Verschwenkung der optischen Achse der spaltförmigen Beleuchtung erlaubt ein Abscannen der Hornhaut entlang der optischen Achse.

Das mit der Spaltlampenanordnung erhaltene Signal hat einen Signalpegel, d.h. die vom Photoempfänger 59 registrierte Strahlungsintensität, dessen Signalverlauf von einem Scanwinkel α der optischen Achse abhängt, auf der die spaltförmige Beleuchtung eingestrahlt wird.

Bei einem Winkel α0 tritt z. B. ein erster Signalpeak auf, der von einem Rückseitenreflex der Hornhaut 14, d.h. vom Reflex am Endothel herrührt. Zwischen einem Winkel α1 und α2 wird z. B. ein Plasmablasenreflex detektiert, dessen Breite ein Maß für die Ausdehnung der Plasmablase 11 entlang der optischen Achse ist. Beim Winkel α3 wird schließlich z. B. ein vom Endothel stammender Vorderseitenreflex registriert. Die bekannte Dicke der Hornhaut 14 skaliert den Abstand zwischen dem Winkel α0 und α3 in ein Dickenmaß, wodurch der Abstand α0 bis α1, d.h. die Höhe der Plasmablase über dem Endothel, der Abstand a1 bis a2, d.h. die Dicke der Plasmablase, sowie der Abstand α2 bis α3, d.h. die Tiefe der Plasmablase unter dem Epithel, in ein Längenmaß umgerechnet werden kann.

Soll zusätzlich zur axialen Information, die durch die Scannbewegung der spaltförmigen Beleuchtung erhalten wurde, eine laterale Information gewonnen werden, kann im Detektionsstrahlengang eine Abbildung auf einen bildgebenden Detektor anstelle des Photoempfängers erfolgen.

Dieses Prinzip betrifft eine Variante der vierten Ausführungsform. Die spaltförmige Beleuchtung fällt nun zentral, d.h. koaxial zum Behandlungsstrahl, ein. Dadurch wird ein Beleuchtungsstrahlengang erhalten, dessen optische Achse mit der optischen Achse des Behandlungsstrahlenganges zusammenfällt. Die Beobachtung erfolgt entlang einer optischen Achse, die schräg zur optischen Achse des Beleuchtungsstrahlenganges liegt. Der Beobachtungsstrahlengang liegt also unter einem Winkel zum Beleuchtungsstrahlengang, wie es auch in der Ausführungsform der Fig. 14 der Fall war, allerdings wird in dieser Bauweise die Scanbewegung des Beleuchtungsstrahlenganges durch eine für den Behandlungsstrahl ohnehin vorgesehene Scaneinrichtung bewirkt. Die von der Scaneinrichtung kommende Strahlung wird noch einmal durch einen Spiegel umgeleitet, der optional als Strahlteiler ausgebildet sein kann und die Beobachtung des Operationsgebietes mit einem Mikroskop ermöglicht.

Die Scaneinrichtung verändert den Einfallswinkel der optischen Achse zum Auge 6, unter dem die Beleuchtungsstrahlung des Beleuchtungsstrahlengangs auf die Hornhaut 14 einfällt. Der Beobachtungsstrahlengang sowie dessen optische Achse sind in der Ausführungsform nicht verstellbar ausgestaltet, wobei dies natürlich zum Gewinnen zusätzlicher Information optional möglich ist. Der Streulichtkanal wird also entlang der (feststehenden) optischen Achse des Beobachtungsstrahlenganges verschoben. Im Beobachtungsstrahlengang ist der Abbildungsoptik ein Bildempfänger nachgeordnet, der ein Bild des Streulichtkanales aufnimmt, der am Schnittpunkt der Achsen liegt.

Eine Variante der vierten Ausführungsform umfasst eine Spaltlampenanordnung, die als Messeinrichtung in einem laserchirurgischen Instrument 1 dient. Diese entspricht im Wesentlichen der soeben geschilderten Bauweise, jedoch wird über den Einkoppelstrahlteiler 4 nun ein Beleuchtungsstrahlengang eingekoppelt, der über das Objektiv eine spaltförmige Beleuchtung entlang der optischen Achse der Behandlungsstrahlung einstahlt. Der Einkoppelstrahlteiler 4 führt die optische Achse der Beleuchtungsstrahlung mit der optischen Achse der Behandlungsstrahlung zusammen.

Dem Einkoppelstrahlteiler 4 ist jedoch eine Beleuchtungsscaneinrichtung vorgeschaltet, die eine eigenständige, von der Behandlungsstrahlung unabhängige Ablenkung der spaltförmigen Beleuchtung ausführt. Damit kann der Streulichtkanal, aus dem durch die spaltförmige Beleuchtung hervorgerufenes Streulicht in den Beobachtungsstrahlengang gelangt, auch lateral gegenüber dem Fokus der Behandlungsstrahlung verstellt werden. Es lässt sich also zu der Information durch das Signal des Bildempfängers zusätzlich noch Information über das Streulichtbild und damit über die Hornhautstruktur seitlich des Fokuspunkts der Behandlungsstrahlung gewinnen.

Bei einer fünften Ausführungsform eines laserchirurgischen Instruments umfasst eine gepulste Laserstrahlquelle mit einer zur Behandlung des betreffenden Gewebes hinreichend großen Pulsenergie, in einer Ablenkeinrichtung, die das von der Laserstrahlquelle kommende Laserstrahlbündel lateral ablenkt, eine durchstimmbare Fokussiereinrichtung, mit der die Lage des Fokuspunktes in der Tiefe des Gewebes eingestellt wird, eine Positioniereinrichtung zur Positionierung des zu behandelnden Gewebes 6 sowie eine Steuereinrichtung zur Ansteuerung der Laserstrahlquelle, der Ablenkeinrichtung und der Fokussiereinrichtung vorgesehen sind. Die Steuereinrichtung steuert die genannten Komponenten an, so dass der Fokus der Laserstrahlung nacheinander auf reale Zielpunkte ZP' mit den Koordinaten (x"', y"', z"') fokussiert wird. Dazu müssen der Steuereinrichtung Informationen über die Koordinaten (x", y", z") von Sollzielpunkten ZP zur Verfügung stehen. Die Positioniereinrichtung kann je nach Anwendung entfallen oder durch eine die Grobausrichtung des Gewebes sicherstellende Einrichtung ersetzt werden.

Weiter sind in diesem laserchirurgischen Instrument ein Energieminderer, ein Detektor und eine Speichereinheit vorgesehen. Die Strahlenergie der gepulsten Laserstrahlquelle wird bei in den Strahlengang eingeschaltetem Energieminderer so weit abgeschwächt, dass der Fokus der Laserstrahlung im Gewebe keine irreversiblen Veränderungen hervorruft. Das von der Laserstrahlquelle abgegebene Laserstrahlbündel kann somit zum einen als Messstrahlenbündel (in einem sogenannten Messregime) als auch als Behandlungsstrahlenbündel (in einem sogenannten Behandlungsregime) in das Gewebe fokussiert gescannt werden. Als Messstrahlenbündel bewirkt das Laserstrahlbündel im realen Messpunkt MP abhängig von den Eigenschaften des Gewebes ein laserinduziertes Signal S, welches von einem Detektor über den (nicht weiter dargestellten) Detektionsstrahlengang empfangen wird. Die detektierten laserstrahlungsinduzierten Signale S werden vom Ausgang des Detektors dem Eingang einer Speichereinheit zugeführt und in der Speichereinheit 79 gemeinsam mit den Koordinaten (x', y', z') der erfassten zugehörigen Messpunkte MP' abgelegt. In einer mit dem Ausgang der Speichereinheit verbundenen Komperatoreinheit werden die laserstrahlungsinduzierten Signale S mit dort abgelegten Schwellwerten Ŝ verglichen.

Dadurch werden diejenigen Messpunkte selektiert, die als Zielpunkte mit dem Behandlungslaserstrahl beaufschlagt werden sollen, wenn der Energieminderer ausgerückt ist. Dadurch wird das Behandlungsregime festgelegt. Die Koordinaten dieser ausgewählten erfassten Messpunkte MP' werden an die Steuereinrichtung weitergeleitet und stehen zur Ansteuerung der Ablenkeinrichtung und der Fokussiereinrichtung zur Verfügung.

Eine Transformation der Koordinaten der erfassten Messpunkte MP' in Koordinaten der Sollzielpunkte ist nicht notwendig, da sie auf dasselbe Koordinatensystem bezogen sind. Das Koordinatensystem (x', y', z') der Messung ist mit dem der Zielpunkte (x"', y"', z"') der Messung sowie dem für die Behandlungs-Laserstrahlung eingestellt identisch bezüglich des Bezugspunktes. Eventuelle Abweichungen von dieser Identität durch gerätespezifische Toleranzen sind nicht weiter störend, da sie auf jeden Fall konstant bleiben und damit ausgeregelt werden können.

Ein erstes Messregime zur Abtastung eines Gewebes lenkt, beginnend von einem Startpunkt, den Laserfokus auf in einem Raster angeordnete Messpunkte. Unterschiedliche Gewebearten, die an einer Grenzschicht aneinanderstoßen, führen zu unterschiedlich laserinduzierten Signalen S. Jedem Messpunkt lässt sich anhand des ausgewerteten Signals S ein Gewebetypus zuordnen. So liegt z. B. ein erster Messpunkt im ersten Gewebetyp und ein zweiter Messpunkt im zweiten Gewebetyp. Daraus lässt sich ein Erwartungsbereich für die mögliche Lage der Grenzschicht ableiten. Die Genauigkeit, mit der die tatsächliche Lage der Grenzschicht durch den Erwartungsbereich angegeben werden kann, hängt vom lateralen Rasterabstand und dem normalen Rasterabstand ab.

Ein anderes Messregime unterscheidet sich gegenüber dem gerade genannten durch eine höhere Tiefenauflösung, d.h. durch einen geringeren normalen Rasterabstand. Die Lage der Grenzschicht lässt sich bei gleichem lateralen Rasterabstand erheblich genauer bestimmen, wenn die Grenzschicht annähernd parallel zur Oberfläche verläuft und keine großen Steigungen aufweist. Je nach vermutetem Verlauf der Grenzschicht kann deshalb durch asymmetrische Gestaltung der Rasterauflösung, beispielsweise durch einseitige Erhöhung in nur einer Dimension die Messgenauigkeit erhöht werden, bzw. durch Verringerung der Rasterabstände in den anderen Dimensionen die Anzahl der Messpunkte und damit die Messdauer verringert werden. Die Abtastung eines Volumens hat den Vorteil, alle dort vorkommenden Strukturen erkennen zu können. So werden z.B. Einschlüsse genauso detektiert, wie die Grenzschicht.

Besteht jedoch nur Interesse an der Lage der Grenzschicht, kann das Messregime zur Verringerung der Messdauer verändert werden, indem eine dritte Variante für ein Messregime mit möglichst wenig Messpunkten und dennoch hoher Auflösung den Verlauf einer Grenzschicht abtastet. Die Abtastung beginnt an einem Startpunkt, von dem bekannt ist, dass er im ersten Gewebetyp liegt. Im vorbestimmten Rasterabstand wird dann der Fokus in Richtung der vermuteten Lage der Grenzschicht verschoben und das Signal S ausgewertet. Überschreitet der Fokus die Grenzschicht, ändert sich das Signal S. Die Tiefenkoordinate der Grenzschicht ist an dieser lateralen Position somit bekannt. Im lateralen Rasterabstand wird dann der Abtastvorgang beginnend in der gleichen Tiefe an einem neuen Startpunkt fortgesetzt. Statt den Fokus aber nun entweder aufwärts oder abwärts zu bewegen, wird alternierend in größer werdendem Abstand vom neuen Startpunkt über und unterhalb der erwarteten Tiefenlage der Grenzschicht gemessen.

Dadurch wird an allen interessierenden lateralen Punkten die Tiefenlage der Grenzschicht bestimmt. Es werden dafür umso weniger Messpunkte benötigt, je besser die vermutete Lage der Grenzschicht an der neuen lateralen Position mit der tatsächlichen Position übereinstimmt. Erfahrungswerte über den Verlauf der Grenzschicht erlauben es also die Anzahl der benötigten Messpunkte weiter abzusenken. Solche Erfahrungswerte liegen beispielsweise für den Verlauf der Bowman'schen Membran vor, die etwa in konstanter Tiefe unter der Hornhautaußenseite liegt und annähernd sphärisch ist. Nach einigen Messpunkten lässt sich daher die vermutete Tiefenlage dieser Membran recht genau vorhersagen, und es sind nur noch wenige Messpunkte nötig, um die exakte Position zu bestimmen.

Auch kann bei vielen Gewebestrukturen von einem gleichmäßigen Verlauf ohne Tiefenschwankungen mit großer Raumfrequenz ausgegangen werden, so dass eine geringere laterale Auflösung von ca. 0,1 mm ausreichend sein kann. Ein Bereich von 10 mm Durchmesser kann dann mit ca. 100.000 Abtastpunkten mit 0,1 mm lateraler Auflösung und 1 µm Tiefenauflösung erfasst werden.

Die geschilderten Ausführungsformen können besonders vorteilhaft für das eingangs erwähnte Operationsverfahren eingesetzt werden. Dazu kann man die Hornhaut des Auges an einem Kontaktglas durch Ansaugen mit hoher Tiefenauflösung (ca. 1 µm) und geringer lateraler Auflösung (z.B. 100 µm) vom Epithel bis zum Endothel über den gesamten Bereich, in dem der chirurgische Eingriff erfolgen soll, vermessen. Bei der fünften Ausführungsform ist dazu der Energieminderer eingeschaltet, und es werden die Schichten der Hornhaut an mehreren Positionen in einem lateralen 100 µm Raster im Wesentlichen senkrecht zur Hornhautoberfläche abgetastet. Im Detektionsstrahlengang wird dann beispielsweise eine Mehrphotonenfluoreszenz bei einer geeigneten Wellenlänge, die auf die Unterschiede der unterschiedlichen Schichten und/oder Grenzschichten sensitiv ist, ortsaufgelöst detektiert. Alternativ kann jedes der zuvor erwähnten Messprinzipien Anwendung finden.

Aus einer Vielzahl derart gewonnener Tiefenprofile lässt sich dann ein dreidimensionales Abbild der Schicht der Hornhaut erstellen. In diesem Abbild lässt sich die lateral aufgelöste Tiefenlage der Bowman'schen Membran erkennen, die je nach Schnittführung bedeutsam sein kann. Zur Behandlung wird dann der Energieminderer aus dem Strahlengang entnommen, so dass bei erneuter Ausführung des Scanvorgangs der erwünschte Schnitt unterhalb der Epithelgrenze ausgeführt wird. Das Epithel bleibt dadurch weitgehend unverletzt, so dass der Schnitt nach einigen Tagen wieder verheilt.

Es kann aber nicht nur das Epithel entlang der Bowman'schen Membran gelöst werden, sondern auch ein tiefer im Stroma liegender Schnitt ist möglich. Die Dicke des am Epithel verbleibenden Stromas kann dabei durch die vorherige Messung genau eingestellt werden, wodurch eine Beschädigung oder Verlust des Epithels ausgeschlossen ist.

## Patentansprüche

1. Vorrichtung zur Behandlung von transparentem Gewebe eines Auges (6) mittels ins Gewebe auf Zielpunkte fokussierter, gepulster Behandlungs-Laserstrahlung (2) mit einer Pulslänge zwischen 1 fs und 100 ps und zur Vermessung des Gewebes (6), mit einer Laserstrahlungsquelle, einer Ablenkeinrichtung (21), einer Fokussiereinrichtung (13) und einer Detektoreinrichtung sowie einer Steuereinrichtung (PC), welche die Laserstrahlungsquelle, die Ablenkeinrichtung (21) und die Fokussiereinrichtung (13) so ansteuert, dass von der Laserstrahlungsquelle abgegebene Beleuchtungs-Laserstrahlung von der Ablenkeinrichtung (21) und der Fokussiereinrichtung (13) nacheinander in mehrere Fokuslagen innerhalb des Gewebes (6) fokussiert wird, wobei die Behandlungs-Laserstrahlung (2) ausgebildet ist, transparente Bereiche des Auges (6), nämlich Hornhaut, Glaskörper und/oder Linse, zu durchdringen und im Fokus optische Durchbrüche im transparenten Bereich zu erzeugen und die Detektoreinrichtung durch die Fokussierung induzierte, gewebespezifische Signale an die Steuereinrichtung (PC) abgibt und die Steuereinrichtung (PC) die Signale Messpunkten zuordnet, deren Ort im Gewebe jeweils durch die Fokuslage
definiert ist, und Messpunkte herausfiltert und dadurch Lagen von Grenzschichten und/oder Einschlüssen im Gewebe bestimmt, wobei die Steuereinrichtung (PC) mittels der herausgefilterten Messpunkte die Zielpunkte für die fokussierte Behandlungs-Laserstrahlung (2) festlegt.

2. Vorrichtung nach Anspruch 1, **dadurch gekennzeichnet, dass** die Behandlungs-Laserstrahlung (2) die Ablenkeinrichtung (21) und die Fokussiereinrichtung (13) durchläuft.

3. Vorrichtung nach einem der Ansprüche 1 oder 2, **dadurch gekennzeichnet, dass** die Laserstrahlungsquelle zur Abgabe der Beleuchtungs-Laserstrahlung und der Behandlungs-Laserstrahlung (2) ausgebildet ist.

4. Vorrichtung nach Anspruch 3, **gekennzeichnet durch** einen Energieminderer, der zeitweise im Strahlengang der Laserstrahlungsquelle nachgeschaltet ist und der die Beleuchtungs-Laserstrahlung abgibt.

5. Vorrichtung nach einem der obigen Ansprüche, **dadurch gekennzeichnet, dass** die Steuereinrichtung (PC) Zielpunkte auch zwischen Messpunkte legt.

6. Vorrichtung nach einem der obigen Ansprüche, **dadurch gekennzeichnet, dass** die Beleuchtungsstrahlung in einen Lichtweg der Behandlungs-Laserstrahlung (2) eingekoppelt ist, wobei eine verstellbare Optik verwendet ist, mit der die Divergenz der Beleuchtungsstrahlung ohne Veränderung der Divergenz der Behandlungs-Laserstrahlung (2) veränderbar ist.

7. Vorrichtung nach einem der obigen Ansprüche, **dadurch gekennzeichnet, dass** die Steuereinrichtung (PC) die Messpunkte in Tiefenrichtung in einem geringeren Rasterabstand anordnet als in lateraler Richtung.

8. Vorrichtung nach einem der obigen Ansprüche, **dadurch gekennzeichnet, dass** die Detektoreinrichtung (3) aus dem Gewebe ausgehende Strahlung interferometrisch detektiert.

9. Vorrichtung nach Anspruch 8, **dadurch gekennzeichnet, dass** die Detektoreinrichtung als optischer Kohärenztomograph (3) aufgebaut ist.

10. Vorrichtung nach einem der Ansprüche 1 bis 7, **dadurch gekennzeichnet, dass** die Detektoreinrichtung aus dem Gewebe ausgehende Strahlung mittels einer konfokalen Abbildung detektiert.

## Claims

1. An apparatus for treatment of transparent tissue of an eye (6) by means of pulsed treatment laser radiation (2) focused to target points in the tissue, pulsed treatment laser radiation (2) having a pulse length of between 1 fs and 100 ps and for measuring the tissue (6), the apparatus comprising a laser radiation source, a deflection device (21), a focusing device (13) and a detector device as well as a control device (PC) which controls the laser radiation source, the deflection device (21) and the focusing device (13) such that illumination laser radiation emitted by the laser radiation source is focused by the deflection device (21) and the focusing device (13) sequentially into several focus positions within the tissue (6), wherein the treatment laser radiation (2) is configured to penetrate transparent regions of the eye (6), namely cornea, vitreous body and/or lens, and to generate optical breakthroughs in the focus in the transparent region and wherein the detector device emits tissue-specific signals induced by the focusing to the control device (PC), and wherein the control device (PC) assigns the signals to points of measurement the location in the tissue of which is defined by the respective focus position, and filters out points of measurement and thus determines positions of boundary layers and/or inclusions in the tissue, wherein the control device (PC) defines the target points for the focused treatment laser radiation (2) on basis of the filtered-out points of measurement.

2. The apparatus according to claim 1, **characterized in that** the treatment laser radiation (2) passes through the deflection device (21) and the focusing device (13).

3. The apparatus according to any of claims 1 or 2, **characterized in that** the source of laser radiation is configured for emitting the illumination laser radiation and the treatment laser radiation (2).

4. The apparatus according to claim 3, **characterized by** an energy reducer, which is, at times, arranged the beam path after the source of laser radiation in and emits said illumination laser radiation.

5. The apparatus according to any of the above claims, **characterized in that** the control device (PC) also positions target points between points of measurement.

6. The apparatus according to any of the above claims, **characterized in that** the illumination radiation is coupled into a light path of the treatment laser radiation (2), wherein adjustable optics are used by which the divergence of the illumination radiation is changeable without changing the divergence of the treatment laser radiation (2).

7. The apparatus according to any of the above claims, **characterized in that** the control device (PC) arranges the points of measurement at a smaller grid spacing in a depth direction than in a lateral direction.

8. The apparatus according to any of the above claims, **characterized in that** the detector device (3) interferometrically detects radiation coming from the tissue.

9. The apparatus according to claim 8, **characterized in that** the detector device (3) is provided in form of an optical coherence tomograph (3).

10. The apparatus according to any of claims 1 to 7, **characterized in that** the detector device detects the radiation emitted by the tissue by means of confocal imaging.

## Revendications

1. Arrangement de traitement du tissu transparent d'un œil (6) au moyen d'un rayonnement laser de traitement (2) pulsé, focalisé sur des points cibles dans le tissu, avec une longueur d'impulsion entre 1 fs et 100 ps, et de mesure du tissu (6), comprenant une source de rayonnement laser, un dispositif de déviation (21), un dispositif de focalisation (13) et un dispositif détecteur ainsi qu'un dispositif de commande (PC), lequel commande la source de rayonnement laser, le dispositif de déviation (21) et le dispositif de focalisation (13) de telle sorte que le rayonnement laser d'éclairage émis par la source de rayonnement laser est focalisé par le dispositif de déviation (21) et le dispositif de focalisation (13) l'un après l'autre en plusieurs positions focales à l'intérieur du tissu (6), le rayonnement laser de traitement (2) étant configuré pour pénétrer à travers les zones transparentes de l'œil (6), à savoir la cornée, le corps vitreux et/ou la lentille et pour générer, au niveau du foyer, des percements optiques dans la zone transparente et le dispositif détecteur délivrant au dispositif de commande (PC) des signaux spécifiques au tissu induits par la focalisation et le dispositif de commande (PC) associant aux signaux des points de mesure dont l'emplacement dans le tissu est respectivement défini par la position focale, et élimine par filtrage les points de mesure et détermine ainsi les positions des couches interfaciales et/ou des inclusions dans le tissu, le dispositif de commande (PC), à l'aide des points de mesure éliminés par filtrage, spécifiant les points cibles pour le rayonnement laser de traitement (2) focalisé.

2. Arrangement selon la revendication 1, **caractérisé en ce que** le rayonnement laser de traitement (2) traverse le dispositif de déviation (21) et le dispositif de focalisation (13).

3. Arrangement selon l'une des revendications 1 et 2, **caractérisé en ce que** la source de rayonnement laser est configurée pour délivrer le rayonnement laser d'éclairage et le rayonnement laser de traitement (2).

4. Arrangement selon la revendication 3, **caractérisé par** un réducteur d'énergie qui est temporairement monté en aval de la source de rayonnement laser dans le trajet de rayon et qui délivre le rayonnement laser d'éclairage.

5. Arrangement selon l'une des revendications précédentes, **caractérisé en ce que** le dispositif de commande (PC) place également des points cibles entre les points de mesure.

6. Arrangement selon l'une des revendications précédentes, **caractérisé en ce que** le rayonnement d'éclairage est injecté dans un trajet de lumière du rayonnement laser de traitement (2), une optique positionnable étant utilisée, laquelle permet de modifier la divergence du rayonnement d'éclairage sans modifier la divergence du rayonnement laser de traitement (2).

7. Arrangement selon l'une des revendications précédentes, **caractérisé en ce que** le dispositif de commande (PC) dispose les points de mesure à un écart de grille plus faible dans la direction de la profondeur que dans la direction latérale.

8. Arrangement selon l'une des revendications précédentes, **caractérisé en ce que** le dispositif détecteur (3) détecte par interférométrie le rayonnement qui émane du tissu.

9. Arrangement selon la revendication 8, **caractérisé en ce que** le dispositif détecteur est réalisé sous la forme d'un tomographe à cohérence optique (3).

10. Arrangement selon l'une des revendications 1 à 7, **caractérisé en ce que** le dispositif détecteur (3) détecte le rayonnement qui émane du tissu au moyen d'une représentation confocale.
